(19) Europäisches Patentamt
European Patent Office
Office européen des brevets

(11) **EP 4 647 510 A1**

(12) **EUROPEAN PATENT APPLICATION**
published in accordance with Art. 153(4) EPC

(43) Date of publication:
**12.11.2025 Bulletin 2025/46**

(21) Application number: **24752811.0**

(22) Date of filing: **02.02.2024**

(51) International Patent Classification (IPC):
***C12Q 1/6874*** *(2018.01)* ***C12Q 1/6806*** *(2018.01)*
***C40B 50/06*** *(2006.01)*

(52) Cooperative Patent Classification (CPC):
**C12Q 1/6806; C12Q 1/6874; C40B 50/06**

(86) International application number:
**PCT/CN2024/075588**

(87) International publication number:
**WO 2024/164956 (15.08.2024 Gazette 2024/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.02.2023 CN 202310122704**

(71) Applicant: **GeneMind Biosciences Company Limited**
**Shenzhen, Guangdong 518023 (CN)**

(72) Inventors:
- **LI, Linsen**
  **Shenzhen, Guangdong 518023 (CN)**
- **SUN, Lei**
  **Shenzhen, Guangdong 518023 (CN)**

(74) Representative: **Michalski Hüttermann & Partner Patentanwälte mbB**
**Kaistraße 16A**
**40221 Düsseldorf (DE)**

(54) **SEQUENCING METHOD**

(57) Provided is a sequencing method. The sequencing method comprises: detecting sequencing signals of a specified position on the surface of a solid-phase support in a sequencing reaction process, the specified position comprising at least two sequencing signals; and on the basis of the difference between the sequencing signals, acquiring the type of the base at the specified position, thereby achieving the sequencing of the specified position. The method achieves the sequencing of multiple signal sites, thus increasing the sequencing speed of the sequencing reaction and the sequencing throughput per unit time.

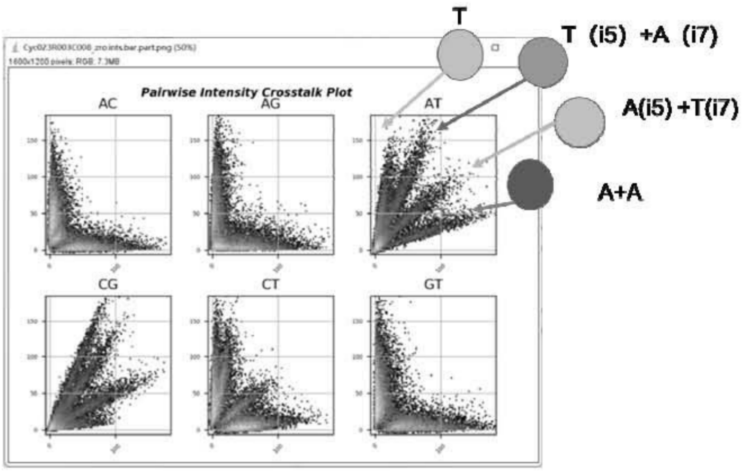

FIG.9



EP 4 647 510 A1

## Description

### FIELD

The present disclosure relates to the field of sequencing, and more specifically to a sequencing method.

### BACKGROUND

Next-Generation Sequencing (NGS), also known as high-throughput sequencing or large-scale parallel sequencing, was first developed from the principle of pyrosequencing. With sequencing by synthesis (SBS) as the basic design concept, NGS takes a target nucleic acid sequences (nucleic acid to be sequenced) as a nucleic acid template and introduces specific nucleotides through a series of base extensions, so that the target nucleic acid sequences is determined based on the type of introduced nucleotides.

Because can both detect a large number of target genes and their variant sites in a single sequencing with high detection sensitivity and specificity for both qualitative and quantitative detection, and relatively low cost for detecting genes and sites for the same number, high-throughput sequencing has shown extremely broad clinical and scientific research application prospects for noninvasive prenatal screening (NIPS), tumor gene mutation, genetic disease, preimplantation genetic screening (PGS) and preimplantationgenetic diagnosis (PGD), pathogenic microorganisms and metagenomics and other fields, becoming the most efficient tool for DNA and RNA sequence analysis at present, and also a supporting technology for research and clinical disease diagnosis and treatment in the era of precision medicine.

However, the current technology of sequencing by synthesis still needs further improvement. For example, current sequencing methods, whether SBS or sequencing by binding (SBB) , can only call one base on a target nucleic acid template at one position in one cycle of sequencing. If two sequencing signals are generated simultaneously at different sites of the target nucleic acid template in one cycle of sequencing, the two sequencing signals will interfere with each other and confuse the signal sources. This limits the speed of sequencing and the data throughput achievable per unit time.

### SUMMARY

The present disclosure aims at solving one of the above technical problems at least to some extent or at least providing a useful commercial option. Therefore, an object of the present disclosure is to provide a method capable of simultaneously obtaining sequencing results of different sequencing templates at one position, i.e., a method capable of simultaneously sequencing multiple different sequencing templates at one position.

In an aspect of the present disclosure, there is provided a sequencing method, including:

detecting sequencing signals at at least one assigned position on a solid support during sequencing, signals of the assigned position at least including two sequencing signals; and
acquiring base types at the assigned position based on a difference between the sequencing signals, thereby achieving sequencing at the assigned position.

According to an embodiment of the present disclosure, this method can simultaneously acquire sequencing signals generated from a plurality of sequencing templates or target nucleic acid sequences at one position which corresponds to a signal point when sequencing signals are collected, or corresponds to a data point when data analysis is performed, and classify these sequencing signals into different sequencing templates or target nucleic acid sequences according to an intensity difference between the sequencing signals, thereby distinguishing multiple signals generated at the same position, thus to obtain sequencing results of multiple sequencing templates or target nucleic acid sequences.

In another aspect of the present disclosure, there is provided a sequencing method, including:

constructing a sequencing library, where the sequencing library includes a plurality of nucleic acid molecules, each nucleic acid molecule includes:

a first barcode, including a first primer hybridization site and a first barcode sequence ligated with a 3' end of the first primer hybridization site;
a second barcode, including a second primer hybridization site and a second barcode sequence ligated with a 3' end of the second primer hybridization site;
a target molecule, ligated between the first barcode and the second barcode, including: a third primer hybridization site and an insert fragment ligated with a 3' end of the third primer hybridization site, the target molecule is ligated with a 3' end of the first barcode via the third primer hybridization site, and the target molecule is ligated with a 5' end of the second barcode via the insert fragment;

amplifying the nucleic acid molecules of the sequencing library after immobilized onto a surface of the solid support, to form a position with a nucleic acid molecule cluster;

introducing a first primer, a blocking molecule and a second primer to hybridize with the nucleic acid molecule of the nucleic acid molecule cluster, where the first primer and the blocking molecule are hybridized to the first primer hybridization site, the second primer is hybridized to the second primer hybridization site, and the blocking molecule is of a non-extendable 3' end;

performing multiple sequencing cycles on the first barcode and the second barcode simultaneously, to acquire sequencing signals in each channel in each cycle of sequencing; and

determining sequences of the first barcode sequence and the second barcode sequence according to an intensity difference between the sequencing signals.

According to an embodiment of the present disclosure, through a single sequencing, adapters at both ends of the sequencing library molecules can be sequenced simultaneously, realizing synchronous sequencing on dual-indexing, which effectively solves the problem of low sequencing efficiency in a condition of the two adapters sequenced separately.

The additional aspects and advantages of the present disclosure will be partially given in the following description, and some will be apparent according to the following description, or be understood through the practice of the present disclosure.

## BRIEF DESCRIPTION OF THE DRAWINGS

The above and/or additional aspects and advantages of the present disclosure will be apparent and readily appreciated from the description of embodiments taken in conjunction with the following drawings, in which:

FIG. 1 is a schematic diagram showing a conventional sequencing;

FIG. 2 is a schematic diagram showing generation of two nucleic acid template amplification clusters with different numbers on a surface of a solid support according to an embodiment of the present disclosure;

FIG. 3 is a schematic diagram showing mixed base signals with different contents according to an embodiment of the present disclosure;

FIG. 4 is a schematic diagram showing a structure of a sequencing library molecule according to an embodiment of the present disclosure;

FIG. 5 shows a process of conventional dual-indexing sequencing according to an embodiment of the present disclosure;

FIG. 6 is a schematic diagram showing a process of dual-indexing sequencing according to an embodiment of the present disclosure;

FIG. 7 is a schematic diagram showing a process of simultaneous sequencing on dual-indexing according to an embodiment of the present disclosure;

FIG. 8 shows a crosstalk plot obtained with control data by hybridizing BC1 alone according to a comparative Example of the present disclosure;

FIG. 9 shows a crosstalk plot obtained by simultaneously sequencing two indexes with BC1 and BC2 respectively according to Example 1 of the present disclosure;

FIG. 10 shows sequencing data statistics according to Example 2 of the present disclosure;

FIG. 11 shows sequencing data statistics according to Example 3 of the present disclosure.

## DETAILED DESCRIPTION

Reference will be made in further detail to the present disclosure in combination with embodiments, to make the technical problems to be solved, the technical solutions and beneficial effects of the present disclosure clearer. It should be understood that the specific embodiments described herein are only used to explain the present disclosure, rather than limit the present disclosure.

The term "at least one" refers to one or more, and "plurality (multiple)" means two or more. "At least one" or expressions similar thereto refers to any combination of these items, including any combination of a single item or a plurality of items. For example, "at least one of a, b, or c" or "at least one of a, b, and c" may mean: a, b, c, a-b (i.e., a and b), a-c, b-c, or a-b-c, where a, b, and c may be single or multiple terms, respectively.

As used in the embodiments of the present disclosure and the appended claims, the singular forms of "a," "an," "the," and "said" are intended to include the plural forms unless the context clearly indicates otherwise.

The terms "first" and "second" are used only for the purpose of description, to distinguish objectives such as locations, objects, etc. from one another, and shall not be understood as indicating or implying relative importance or implicitly indicating the number of technical features indicated. For example, a first primer may also be referred to as a

second primer, and a second primer may also be referred to as a first primer, without departing from the scope of embodiments of the present disclosure. Thus, a feature defined by "first" and "second" may explicitly or implicitly include one or more of such features.

In the description of the present disclosure, a concentration or content of the relevant components or substances mentioned may not only refer to the specific content of each component or substance, but also indicate a proportional relationship between the contents of each component or substance. Hence, the content of the relevant components, scaled up or reduced according to the description of the embodiment of present disclosure, are also within the scope disclosed in the description of the embodiment of the disclosure. The content may be mass percentage content, molar concentration percentage content, etc., and the percentage content may be a specific percentage content, or may be a relative percentage content determined by taking the content of a certain reference substance as a standard.

It should be understood that in various embodiments of the present disclosure, the sequence number of each process does not mean the execution sequence, where some or all of the steps may be executed in parallel or sequentially, and the execution sequence of each process should be determined by its function and internal logic, and should not constitute any limitation on the implementation process of the embodiments of the present disclosure.

In embodiments of the present disclosure, the term "sequencing" may also be referred to as "nucleic acid sequencing" or "gene sequencing", which may be interchangeable in terms of expression, and all refer to the determination of the type and arrangement order of bases or nucleotides, including nucleotide analogs, in nucleic acid molecules. Sequencing as it is called involves the process of nucleotides binding to a template and collecting the corresponding signals generated by the nucleotides, including analogs. Sequencing involves sequencing by synthesis (SBS) and/or sequencing by ligation (SBL), including DNA sequencing and/or RNA sequencing, and long fragment sequencing and/or short fragment sequencing. Long fragments and short fragments as they are called are relative, for example, nucleic acid molecules longer than 1 Kb, 2 Kb, 5 Kb or 10 Kb may be referred to as long fragments, while nucleic acid molecules shorter than 1 Kb or 800 bp may be called short fragments.

The sequencing generally involves multiple cycles of process to achieve the determination of the types and sequence of multiple bases or nucleotides on the nucleic acid template. The examples of the present application refer to each cycle of the "process to achieve the determination of the types and sequence of multiple bases or nucleotides on the nucleic acid template" as one "cycle of sequencing". The "cycle of sequencing", also known as "sequencing cycle", may be defined as the completion of one base extension of the four types of nucleotides/bases; in other words, one "cycle of sequencing" may be defined as the determination of the base or nucleotide type at any given position on the template. For sequencing platforms that achieve sequencing on the basis of polymerization or ligation reactions, one cycle of sequencing includes the process of binding four types of nucleotides (including nucleotide analogs) to the nucleic acid template at a time according to the base complementary rule and acquiring the corresponding signals emitted. For platforms that achieve sequencing on the basis of the polymerization reaction, a reaction system includes reaction substrate nucleotides, polymerase, and a nucleic acid template. A sequence fragment (a sequencing primer) binds to the nucleic acid template, and on the basis of the base pairing rules and the principle of polymerization reaction, the added reaction substrate nucleotides are linked to the sequencing primer under the catalysis of the polymerase to achieve the binding of the nucleotide to a specific position on the nucleic acid template. Generally, one cycle of sequencing may include one or more base extensions (repeats). For example, four types of nucleotides are sequentially added to the reaction system to each perform base extensions and corresponding acquisition of reaction signals, and one cycle of sequencing includes four base extensions; for another example, four types of nucleotides are added into the reaction system in any combinations (such as in pairs or in one-three combinations), the two combinations each perform base extensions and corresponding acquisition of reaction signals, and one cycle of sequencing includes two base extensions; for yet another example, four types of nucleotides are added simultaneously to the reaction system for base extension and reaction signal acquisition, and one cycle of sequencing includes one base extension.

The term "nucleic acid molecule" refers to a polymeric form of nucleotides of any length, and may include ribonucleotides or analogs thereof, deoxyribonucleotides or analogs thereof, and mixtures thereof. The nucleic acid molecule may be referred to as a single-stranded polynucleotide or a double-stranded polynucleotide. Nucleotides in a nucleic acid molecule may include naturally occurring nucleotides and functionally alternative analogs thereof. Examples of the analogs may hybridize to the nucleic acid in a sequence-specific manner, or may be used as templates for replication of a specific nucleotide sequence. Naturally occurring nucleotides typically have a backbone including phosphate diester bonds. The analog may be of a structure with alternative backbone linkages including any of a variety of linkage known in the art. Naturally occurring nucleotides typically have deoxyribose (e.g., as found in DNA) or ribose (e.g., as found in RNA). The analog may be of a structure with substituted sugar moieties, including any of those known in the art. Nucleotides may include natural bases or non-natural bases. Bases in natural DNA may include one or more of adenine, thymine, cytosine, and/or guanine, and bases of the natural RNA may include one or more of adenine, uracil, cytosine, and/or guanine. Nucleotides may also include any non-natural base or analog thereof, such as locked nucleic acids (LNAs) and bridging nucleic acids (BNAs).

The term "nucleic acid template" refers to an original nucleic acid molecule or a fragment thereof that is bound

with nucleotides or nucleotide analogs by successive cycles of base extension. The nucleic acid template may be the entire sequence of a nucleic acid molecule or a partial fragment thereof. The nucleic acid template may be a nucleic acid fragment that is used as a template in an extension reaction during sequencing by synthesis, where since the bases added in the extension reaction satisfy the base-pairing principle with the sequencing template, the sequence of the sequencing template could be determined by detecting the type of bases added in each cycle of extension. The sequence of the nucleic acid template herein may generally include, but is not limited to, at least one of a sequence of a target molecule, a UMI sequence, a sample barcode sequence, and is sometimes referred to herein as an "insert fragment" or a "target molecule".

The term "cluster" refers to a plurality of nucleic acid molecules generally included at one position on a solid support, such as a chip, but usually the plurality of nucleic acid molecules at this position are derived from the same nucleic acid molecule, for example, an amplified cluster including nucleic acid molecules formed by performing bridge PCR on the same nucleic acid molecule.

The term "primer" refers to an oligonucleotide or nucleic acid molecule that may hybridize to a target sequence of interest. In embodiments, the primer functions as a substrate onto which nucleotides may be polymerized by polymerase. For example, primers may be used as starting points for DNA or RNA synthesis. For example, a sequencing primer may hybridize to a synthesized nucleic acid template strand, so as to initiate a synthesis of a new strand complementary to the synthesized nucleic acid template strand. Primers may include any combination of nucleotides or analogs thereof. In some examples, the primer is a single-stranded oligonucleotide or polynucleotide.

The term "probe" includes at least a "primer", e.g., a probe may be functionally identical to a primer, being an oligonucleotide or nucleic acid molecule that hybridizes to a target sequence of interest. In this case, "probe" and "primer" may be interchanged. In addition, the probe may also include other parts, such as other nucleic acid fragments, proteins, small molecules, etc. linked to primers.

The term "target nucleic acid sequences" or "nucleic acid template" may refer to a nucleic acid sequence to be analyzed.

The term "library" in embodiments of the present disclosure refers to a pool/set of nucleic acid molecules including multiple target nucleic acid fragments/nucleic acid fragments to be sequenced derived from a nucleic acid sample to be sequenced. In general, multiple target fragments/fragments to be sequenced are treated, e. g., to ligate known sequences to one or both end of the target fragment, such as adding adaptors (sequencing adaptors), so that the library could be linked or immobilized onto chips thus suitable for loading onto sequencing platforms for sequencing.

The term "channel" in embodiments of the present disclosure refers to a channel formed in different ways and capable of screening and distinguishing four bases from A, C, G and T during the sequencing. The "channel" referred to in embodiments of the present disclosure includes a four-channel condition, which refers to four optical channels formed by using different excitation lights, different fluorescence color filters, etc. in the photographing during sequencing, which are capable of screening and distinguishing fluorescence signals of four fluorescent bases from A, C, G and T/U (indicating T or U). In actual sequencing, photographs are taken in four different fluorescence channels to form images. Ideally, each fluorescence channel only has the fluorescence signal of the corresponding fluorescent base type, but in practice, due to the influence of fluorescence crosstalk, fluorescence signals of other bases will also appear in each channel in addition to the fluorescence signal of the corresponding fluorescent base. The "channel" referred to in the embodiment of the present disclosure also includes a dual-channel condition, which refers to optical channels formed by using different excitation lights, different fluorescence color filters, etc., screening and distinguishing fluorescence signals of two fluorescent bases from A, C, G and T/U each time, and obtains, through two times, fluorescence signals of four fluorescent bases A, C, G and T/U. The "channel" referred to in the embodiment of the present disclosure also includes a single-channel condition, which refers to an optical channel formed by using different excitation lights, different fluorescence color filters, etc., screening and distinguishing fluorescence signals of one fluorescent bases from A, C, G and T/U each time, and obtains, through four times, fluorescence signals of four fluorescent bases A, C, G and T/U. Of course, the number of channels is not limited by the above.

The term "crosstalk", "laser-crosstalk" or "spectra-crosstalk", also known as "spectral crosstalk" or "spectral crossover" in embodiments of the present disclosure refers to a signal corresponding to one base diffusing into a signal corresponding to another base. For sequencing platforms that use differently labeled fluorescent molecules to call different bases, if emission spectra of two or more selected fluorescent molecules overlap, it is possible to detect signal diffusion of one fluorescent molecule into another fluorescent channel in one cycle of sequencing.

The term "chip" in embodiments of the present disclosure refers to a solid support having a surface, e. g., a planar surface, to which biomolecule to be sequenced are attached. It is also referred to as a sequencing chip or flow cell.

The term "ribosyl" refers to a radical group formed by removing one or more hydrogen atoms from ribose. Ribose is of a chemical formula of $C_5H_{10}O_5$, and has two configurations: L-ribose and D-ribose. The chemical structure of L-ribose is shown as follows:

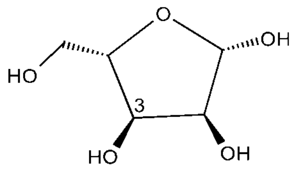.

The chemical structure of D-ribose is shown as follows:

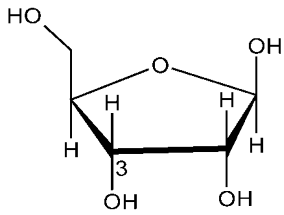.

The term "deoxyribosyl" refers to a radical group of deoxyribose with one or more hydrogen atom removed. Deoxyribose is also known as D-deoxyribose, 2-deoxy-D-ribose, thyminose. Deoxyribose has the chemical formula $C_4H_9O_3CHO$ ($C_3H_{10}O_4$), and its chemical structure is shown below:

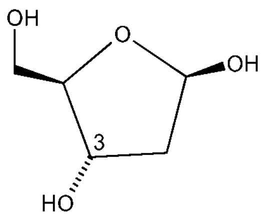.

In current high-throughput sequencing, in order to obtain identifiable signals and improve data availability, when collecting signals from a position or a nucleic acid cluster, bright spots ( signal spots) formed at the same position containing only one type of base signal are generally used. That is, as shown in FIG. 1, existing sequencing methods usually perform identical sequencing reactions across all clusters, with the same nucleotide(s) being incorporated in every cycle. In other words, the existing base sequencing methods aims to ensure that one signal spot contains sequencing signal corresponding to only one type of base. If two or more base signals exist in a bright spot in an image collected from a sequencing cycle, such a spot is generally called a multi-signal spot. Multiple signals would interfere with judgment to the type of introduced base, thus reducing the accuracy of base calling. Therefore, in conventional sequencing, it is usually expected that each bright spot contains only one sequencing signal corresponding to only one type of base, that is, each bright spot corresponds to a single signal, and such multiple signal points need to be avoided as much as possible. However, conventional sequencing has certain limitations that limit its use in applications where multiple signal spots are generated.

For example, a sequencing cycle can only call one base of a target nucleic acid template at one position, which limits the speed of sequencing and the data throughput achievable per unit time. Based on this problem in existing sequencing, the inventors consider that if multiple sequencing signals formed at one position can be called simultaneously, then one nucleic acid molecule could be designed as several target nucleic acid sequences for synchronous sequencing, so that multiple target nucleic acid sequences derived from said one nucleic acid molecule can be sequenced at one time, thus increasing the sequencing speed by multiple times.

For another example, in actual sequencing, when a library is immobilized on the surface of the solid support, nucleic acid molecules in the library will hybridize with probes immobilized on the surface of the solid support and thus be randomly immobilized at a certain position. In most cases, one nucleic acid molecule is immobilized at one position, in which the nucleic acid molecule are amplified to form an amplification cluster. However, in some occasional case, one position may be doped with small amount of other nucleic acid molecules, causing amplification clusters containing copy sets of two nucleic acid molecules, resulting in multi-molecule signals at one position. Illustratively, in the case of the solid support is an array chip, copy sets of two nucleic acid molecules are formed within a nanowell. In images formed in sequencing, the bright spot corresponding to this position correlates with two nucleic acid molecules. Since two sequencing signals formed at the same position in a sequencing cycle cannot be effectively called, in order to reduce the impact of such multi-molecule signals on sequencing accuracy, the data at this position will be discarded in the subsequent data analysis, leading to a reduced amount of sequencing valid data, thereby decreasing sequencing

throughput.

Based on this, the inventors of the present disclosure provide a novel sequencing method capable of simultaneously calling identical or different bases in two different nucleic acid templates or target nucleic acid sequences in each cycle of extension at one position. According to this method, the sequencing is synchronously performed on a plurality of nucleic acid templates or target nucleic acid sequences at the same position, and sequencing signals generated by different sequencing templates or target nucleic acid sequences are of different intensities, thus to distinguish and classify different sequencing signal data subsequently. That is, in a position containing a plurality of nucleic acid templates or target nucleic acid sequences, multiple different nucleic acid templates or target nucleic acid sequences are simultaneously sequenced, and the multiple different nucleic acid templates or target nucleic acid sequences generate sequencing signals with different intensities. Therefore, multiple sequencing signals generated at the same position in one cycle of base extension may be retained, and sequencing data corresponding to these sequencing signals can still be effectively utilized to obtain sequencing results of multiple nucleic acid templates, thereby improving the sequencing speed and sequencing throughput per unit time. Thus, according to the method of the present disclosure, a plurality of different nucleic acid templates or target nucleic acid sequences can be designed in one position.

The method includes: detecting sequencing signals at an assigned position on a solid support during sequencing, the assigned position at least including two sequencing signals; and acquiring base types at the assigned position based on a difference between the sequencing signals, thereby achieving sequencing at the assigned position.

In embodiments of the present disclosure, a solid support is understood as a carrier or support for immobilizing nucleic acid molecules or nucleic acid templates. In some application examples, the solid support may also be referred to as a solid substrate, sequencing chip, or sequencing biochip. In an embodiment, the solid support is a substrate having an array of micropores, and each position corresponds to a position where a micropore is located. An exemplary solid support is a nanowell pattern chip including an array arrangement of nanowells, which may also be referred to as nanopores, micropores or pores. In embodiments of the present disclosure, the nanowell and nanopore referred to herein may be exchangeable for each other, and refer to a pore structure with nanoscale size formed on the surface of a solid support. The shape of the nanowell or nanopore is not strictly limited and may be circular, elliptical, triangular, quadrilateral or other polygonal, or even no particular shape. To facilitate uniformity of nucleic acid template amplification, in some embodiments, the nanowell or nanopore is arranged in the shape of a symmetrical pattern in cross section, exemplarily, the cross section of the nanowell or nanopore is circular or regular polygon, and the more sides of the regular polygon, the more favorable it is for smooth extension of the nucleic acid template amplification. Examples of pattern chips include pattern chips from Illumina Inc. and DNB (DNA nanoball) pattern chips from MGI. In an embodiment, the solid support is a planar substrate, the surface of which includes a plurality of surface-treated sites, one site corresponding to a position. Examples of such solid support are such as random chips.

The surface of the solid support is immobilized with nucleic acid templates, and the nucleic acid templates are linked to the surface of the solid support. In one embodiment, the surface of the solid support, via a covalent bond to a molecule/group on the surface thereof, is attached with probes, and the nucleic acid template is attached to the surface of the solid support via the probe. The probe is an oligonucleotide or nucleic acid molecule fragment capable of hybridizing to a target nucleic acid molecule of interest. In an embodiment, at least a portion of the probe is configured to hybridize to at least a portion of a 3' end of the nucleic acid template. In another embodiment, one end of the nucleic acid template is covalently bonded to a molecule/group on the surface of the solid support, thereby attaching to the surface of the solid support.

In this embodiment, the nucleic acid template is immobilized at a specific position on the solid support. In embodiments of the present disclosure, a position on the surface of the solid support may be understood as a point on the surface of the solid support for immobilizing the amplification cluster of nucleic acid molecules, and there is a certain distance between two adjacent such points, and this distance is sufficiently larger than a distance between nucleic acid molecules at the same position, such that sequencing signals generated in the two points can be distinguished on the image. Illustratively, each nanowell is a position on the surface of the nanowell pattern chip. In embodiments of the present disclosure, the solid support may be designed to determine distribution or arrangement of positions. In an embodiment, for a non-nanowell pattern chip, the position may be determined by forming dot probe clusters on the surface of a solid support. Similarly, a probe cluster refers to a plurality of probes clustered within a single point on the surface of a solid support, and there is a certain distance between two adjacent probe clusters, and this distance is sufficiently greater than a distance between probes in the same probe cluster, such that sequencing signals generated at the points where different probe clusters are located could be distinguished when sequencing are performed on nucleic acid templates to which probes are linked.

In embodiments of the present disclosure, ideally, one nucleic acid template is immobilized in a hybridization manner at each position, to form a cluster composed of a plurality of identical nucleic acid templates after amplification. In some unavoidable cases, there may be conditions where there are very few positions with no nucleic acid template immobilized or only one nucleic acid template immobilized at one position.

Conventionally, in the sequencing signals obtained by one cycle of base extension, two or more different

sequencing signals appearing at one position indicates that there may be two or more different nucleic acid templates or two or more different target nucleic acid sequences from the same nucleic acid template. In order to improve sequencing accuracy, sequencing signals at these positions will be not counted, and the corresponding sequencing data will be removed. Different from the expectation of sequencing only one nucleic acid template or one target nucleic acid sequence at one position in the past, in embodiments of the present disclosure, two or more different nucleic acid templates or different target nucleic acid sequences are allowed to coexist at some positions, and the sequencing method provided by embodiments of the present disclosure includes sequencing different nucleic acid templates or different target nucleic acid sequences in these positions. Because the position containing two or more different nucleic acid templates or different target nucleic acid sequences will generate two or more signals in one cycle of sequencing, this corresponding position may also be called a multi-signal position.

It should be noted that, in the position where multiple signals are generated, the nucleic acid templates or the target nucleic acid sequences may be provided in various ways. According to embodiments of the present disclosure, different sequencing templates in a multi-signal position may be generated "accidentally" or "actively" at the same position. For example, multiple different nucleic acid templates may be provided at one position, so as to form a multiple signal position when the nucleic acid templates at this position are sequenced. Alternatively, respective primer hybridization sites may be arranged at a plurality of the same nucleic acid templates, such that the nucleic acid templates at this position generate multiple different target nucleic acid sequences, so as to form a multiple signal position when the nucleic acid templates at this position are sequenced. Theoretically, it is feasible as long as multiple sequencing signals could be generated at the same position during the sequencing.

In embodiments of the present disclosure, the multi-signal position includes two implementation cases. In a first implementation case, there are multiple different nucleic acid templates at one position, and nucleic acid templates in the position are simultaneously sequenced, thereby realizing simultaneous sequencing on the multiple nucleic acid templates. In a second implementation case, there are identical nucleic acid templates at one position, and these identical nucleic acid templates are designed with multiple target nucleic acid sequences, which are simultaneously sequenced, thereby realizing simultaneous sequencing on the multiple target nucleic acid sequences. Sequencing methods are described below in combination with the two implementation cases.

For the first implementation case, there are multiple different nucleic acid templates at one position, and the multiple different nucleic acid templates are simultaneously sequenced.

In the first implementation case, the surface of the solid support includes a class of positions which include n different nucleic acid templates, such that at the same one position, among different sequences (i.e. target nucleic acid sequences) at least two of them generate different sequencing signals in at least one cycle of sequencing. For ease of description and distinction, such a position is referred to simply as "a first position". It should be understood that the first position contains two or more nucleic acid templates, but this does not mean that each first position has the same number of nucleic acid templates. Illustratively, at some first positions there are two different nucleic acid templates; at other first positions there are three different nucleic acid templates; and at still other first positions there are four or more (e.g., four, five, or more) different nucleic acid templates. Certainly, the case that all the first positions contain the same number of nucleic acid templates is also within the scope of the present disclosure, and could be also realized with the determination of the base or nucleotide type and sequencing on the nucleic acid template through the sequencing method provided by the present disclosure.

In some embodiments, at the first position, the n nucleic acid templates are of different copy numbers. Accordingly, in one cycle of sequencing, the sequencing signals obtained in dominant base signal channels for each nucleic acid template can be distinguished, thus realizing assignment of the sequencing signal to each base template. In some embodiments of the first implementation case, the first position may be obtained by chance upon hybridization of the library molecules to the surface of the solid support. Illustratively, taking a pattern chip as an example, in occasional circumstances, two nucleic acid templates are hybridized in a nanowell. Generally, compared to one hybridized later or hybridized to the edge region of the nanowell, a nucleic acid template firstly hybridized to the surface of the solid support or hybridized to the central region of the nanowell present amplification advantages, where higher amplification multiples are prone to occur during amplification. The difference in amplification multiples results in differences in sequencing signals between the two nucleic acid templates, such as intensity differences.

Certainly, the n nucleic acid templates at the first position may also be obtained by other means. In some embodiments, the n nucleic acid templates at the first position are obtained by: providing the solid support, binding an surface thereon with n probes having different sequences, where the n probes have different amounts; amplifying hybrids obtained by hybridizing the n different nucleic acid templates onto the surface of the solid support, where the n different nucleic acid templates hybridize with the n different probes respectively, such that 2 to n different nucleic acid templates bind to one position with different amounts, and designating this one position as the first position.

Illustratively, referring to FIG. 2, the surface of the solid support is bound with two combinations probes, a probe set 1 and a probe set 2, in fixed proportions. For example, two probes in probe set 1 may perform bridge amplification on a first target nucleic acid molecule, while two probes in probe set 2 may form bridge amplification with a second target nucleic

acid molecule. In other cases, probe set 1 and probe set 2 may each have only one probe, and that probe may separately perform rolling circle amplification with the first target nucleic acid molecule and the second target nucleic acid molecule. After the target library (target nucleic acid molecules) are captured, amplified and hybridized with sequencing primers, amplification clusters containing the first target nucleic acid molecules and the second target nucleic acid molecules are formed on the surface of the solid support. Since the number of each probe has been designed to a specific proportion, the number of copies of the first target nucleic acid molecule and the second target nucleic acid molecule in the amplification clusters is correspondingly in a fixed ratio, such as 1:2, 1:3, 1:4, etc., but not limited to the enumerated cases. The amplification clusters thus obtained may be regarded as the first position. In base extension with two sequencing primers that are hybridized to the two target nucleic acid molecules respectively, when the two target nucleic acid molecules are introduced with different base types, the corresponding base types could be assigned to the sequencing of the two different nucleic acid molecules according to intensities of the two signals detected each time. For example, when the number of the first target nucleic acid molecule to that of the second target nucleic acid molecule in the amplification cluster is of 1:2 and the bases introduced into the two target nucleic acid molecules are T and A, respectively, the intensities of two detected signals also tend to be 1:2.

In embodiments of the present disclosure, n probes at the first position differ in quantity or concentration. In some embodiments, among the n different probes at the first position, a difference in the number between different probes is at least greater than or equal to 10% of the total number the n probes, a difference in a molar concentration between different probes is at least greater than or equal to 10% of a total molar concentration of the n probes, and more preferably, the difference in the number between different probes is at least greater than or equal to 20% of the total number of the n probes, or the difference in the molar concentration between different probes is at least greater than or equal to 20% of the total molar concentration of the n probes. It should be understood that, in embodiments of the present disclosure, different probes have the same difference standard, for example, taking the total number of the n probes as the standard, the difference among different probes is quantity difference; similarly, taking the total molar concentration of the n probes as the standard, the difference among different probes is molar concentration difference.

In one embodiment, the n different nucleic acid templates include multiple sequence units to be sequenced with different numbers, identical nucleic acid templates have the same sequences of the sequence units to be sequenced, and different nucleic acid templates have different sequences of the sequence units to be sequenced. At this point, in a sequencing cycle, different nucleic acid templates have different numbers of base extension sites, thereby generating signals with different intensities. For this embodiment, since the difference in signal intensity is contributed by the number of sequence units to be sequenced which constitute the nucleic acid template, n nucleic acid templates have the same or different copy numbers at the first position in this case. In this embodiment, there are differences in the number of sequence units to be sequenced in different nucleic acid templates. In some embodiments, the difference in the number of sequence units to be sequenced among the n different nucleic acid templates is at least greater than or equal to 10% of the total number of sequence units to be sequenced. More preferably, the difference in the number of sequence units to be sequenced among the n different nucleic acid templates is at least greater than or equal to 20% of the total number of sequence units to be sequenced. In order to enable all sequence units to be sequenced of nucleic acid templates to be sequenced and improve sequencing throughput, a primer binding site may be provided for each sequence unit to be sequenced in n different nucleic acid templates.

In embodiments of the present disclosure, there is no strict requirement for the copy number difference of n nucleic acid templates. In some embodiments, if the copy number of the nucleic acid template with the highest copy number is 100%, the copy number differences among the n nucleic acid templates are between 5% and 95%, where all sequencing signal differences generated by the multiple nucleic acid templates in the sequencing could be distinguished based on the number difference of the nucleic acid templates, thereby realizing the sequencing of multiple signal positions. In some embodiments, if the copy number of the nucleic acid template with the highest copy number is 100%, the copy number differences among the n nucleic acid templates are between 20% and 80%. Illustratively, when n=2, a ratio of copy numbers of the two nucleic acid templates may be 10:8, 10:7, 10:6, 10:5, 10:4, 10:3, 10:2, etc. It should be understood that it is unable to count the copy numbers of each nucleic acid template at the first position, the percentage content or ratio herein is therefore only used to reflect a trend of copy numbers of different nucleic acid templates, and the actual ratio may fluctuate to a certain extent on this basis. In theory, it is feasible as long as the difference in the amount of the two is sufficient to recognize the difference in sequencing signals generated by the sequencing.

In embodiments of the present disclosure, n is an integer greater than or equal to 2, i.e., there are two or more nucleic acid templates at one first position. In addition, there is not particularly limited to the types of nucleic acid templates provided at one position, which may be two, three, four or more as long as the signal intensities could be distinguished. It should be understood that the greater the number of nucleic acid templates at one first position, the more complex the corresponding sequencing signals generated during the sequencing, the more complicated algorithm for analyzing the sequencing data corresponding to the sequencing signals and realizing the assignment of each sequencing signal. In some embodiments, n is selected from 2 to 4, illustratively n is 2, 3, or 4. In this case, the number of nucleic acid templates at one first position is controlled within 4. A relative content of each nucleic acid template at the first position is judged

according to the sequencing signals collected by each channel in one or more cycles of sequencing and the intensities of the sequencing signals in each channel. In an embodiment, different sequenced templates at the same one first position generate different sequencing signals in at least one cycle of sequencing, and in this case, the relative content of each nucleic acid template at the first position could be determined according to the sequencing signal intensity generated in this cycle of sequencing. In another embodiment, different sequenced templates generate the same portion of sequencing signals in one cycle of sequencing, and in this case, the relative contents of nucleic acid templates with different sequencing signals at the first position could be determined through one cycle of sequencing. The remaining nucleic acid templates whose relative content has not been determined will generate different sequencing signals during other cycles of sequencing, and in this case, their relative content can be determined by identifying the intensity of sequencing signals based on the same method. With two or more cycles of such base extension, the content of each nucleic acid template at one first position could be determined. On this basis, the base or nucleotide type introduced onto each nucleic acid template in the current cycle of sequencing could be determined based on the relative content of each nucleic acid template at the first position and the sequencing signal intensity generated in each cycle of sequencing. Even if two nucleic acid templates produce the same sequencing signals, the sequencing signal may also be assigned based on the fixed content of each nucleic acid template. That is, for the first position containing a plurality of different nucleic acid templates, the base or nucleotide type of each cycle of sequencing could be called according to signal characteristics, and finally, according to sequencing signals such as collected fluorescent images obtained by multiple cycles of sequencing, sequences of the plurality of nucleic acid templates could be identified from one first position.

Illustratively, a pattern chip is taken as an example to illustrate an identification to sequences of two different nucleic acid templates at one first position. In principle, there may be dimolecular signal points formed by two different nucleic acid template amplification clusters in a nanowell on the pattern chip. Due to the difference in amplification order and amplification efficiency, the amplification quantity ratio of the two nucleic acid fragments is 7:3. Then, the signal intensities generated by the two nucleic acid fragments per cycle of base extension in a certain two-base channel or twice in one channel also shows a ratio of 7:3. Based on the intensity characteristics of the sequencing signal obtained by each cycle of base extension, the base or nucleotide type introduced onto each of the two nucleic acid templates in each cycle of base extension could be known.

According to embodiments of the present disclosure, different nucleic acid templates are provided with distinct primer hybridization sites, which are also referred to as primer recognition sites. Primer hybridization sites, i.e., first sites, exist n primer hybridization sites.

Exemplary sequencing methods of a first embodiment include the following steps:

S11: Detecting sequencing signals at the first position of the solid support during sequencing.

With solid support obtained, multiple sequencing cycles are performed on the solid support, and the sequencing signals, at an assigned position, in each base-signal channel are acquired in sequencing. This step includes: performing multiple sequencing cycles on the nucleic acid templates at the first position, to acquire the sequencing signals in each channel for the first position.

During sequencing, for each nucleic acid template, each cycle of sequencing extends a nucleotide or analog thereof conjugated with a specific signal group (usually a fluorescent group). By detecting the sequencing signal (usually a fluorescent signal) generated by the specific signal group in a specific channel, the base type or nucleotide introduced into the sequencing and thus the sequence of the nucleic acid template can be determined. It should be understood that, in embodiments of the present disclosure, sequencing signals of four bases may be obtained simultaneously through a four-base channel, or be performed through a dual-base channel with twice signal acquisitions, or be performed through a single-base channel with four times of signal acquisitions.

According to embodiments of the present disclosure, there are n different nucleic acid templates at the first position, at least part of the n different nucleic acid templates generate different sequencing signals in at least one cycle of sequencing. Illustratively, at least two of the n different nucleic acid templates generate different sequencing signals in at least one cycle of sequencing. In this case, the relative content of the at least two of nucleic acid templates each at the first position could be determined according to the sequencing signal intensity generated in this cycle of sequencing. The remaining nucleic acid templates whose relative content has not been determined will generate different sequencing signals during other cycles of sequencing, and in this case, their relative content can be determined by identifying the intensities of sequencing signals based on the same method. With two or more cycles of such base extension, the content of each nucleic acid template at one first position could be determined. On this basis, the base or nucleotide type introduced onto each nucleic acid template in the current cycle of sequencing could be determined based on the relative content of each nucleic acid template at the first position and the sequencing signal intensity generated in each cycle of sequencing. Even if two nucleic acid templates produce the same sequencing signals, the sequencing signal may also be assigned based on the fixed content of each nucleic acid template.

In embodiments of the present disclosure, in sequencing, acquiring the sequencing signal of each channel obtained at the first position includes obtaining the sequencing signal of each channel in each cycle of sequencing. In some embodiments, the sequencing signal is an optical signal. Illustratively, the sequencing signal is a fluorescent signal

generated by a fluorescent group on a nucleotide, and in this case, acquiring the sequencing signal of each channel includes: acquiring a fluorescent image of each base channel after a cycle of base extension, and acquiring a bright spot signal in the fluorescent image of each channel. A bright spot in the fluorescent image corresponds to a signal of a position. Correspondingly, the signal generated at the first position is a multi-signal, and in this case, the corresponding bright spot is a bright spot formed by multiple signals mixed. In some embodiments, acquiring the bright spot signal in the fluorescent image of each channel includes determining fluorescence intensity of each bright spot in the fluorescent image. Specifically, the fluorescent image may be extracted with a gray scale value, and the fluorescence intensity of each bright spot in the fluorescent image may be confirmed after performing a correction to the gray scale image obtained. Exemplary corrections include crosstalk correction and/or phase imbalance correction, including phasing and prephasing correction.

Since multiple different signals are generated at the first position containing a plurality of nucleic acid templates during each cycle of sequencing and there may be crosstalk among these signals, the correction is performed on sequencing signal data to eliminate interference after obtaining the sequencing signal data of the nucleic acid template cluster, according to an embodiment of the present disclosure. This procedure can further improve the accuracy of sequencing.

In some embodiments, the crosstalk correction is performed to eliminate crosstalk between channels, the main purpose of which is to ensure that each fluorophore only displays a signal in a single channel to facilitate calling of the true base signal in the position. According to embodiments of the present disclosure, there are 12 permutations and combinations in total for performing pairwise correction on 4-dimensional data according to permutations and combinations, for example, AC correction indicates correction of A signal by using C, AT indicates correction of T with A, and TA indicates correction of A with T. In one embodiment, the correction to dual-channel is as follows: for a specific combination, selecting a combination to form x, y and calculate the crosstalk correction of x versus y. specifically, selecting a fixed brightness segment in x, and for (x,y) in this interval, taking the lower edge of a (x,y) scatterplot according to the fixed x step size. The discrete points thus obtained represent the minimum crosstalk of x versus y. Next, a slope of a linear fit may be obtained by performing the linear fit on the sampled set of discrete points, and with the slope k of the linear fit, correction calculation, e.g. TA correction, is performed, to correct T signal with A:

$$T' = T - A \times k.$$

According to some specific embodiments, it can be found that after correction, an angle between the A arm and the abscissa axis on the AT graph can become 0.

In addition, according to embodiments of the present disclosure, as cycles of extension grow, the inventors found that the signal of each base is not purely single after multiple cycles of extension. This is due to lead and lag in chemical reactions, resulting in phase error, which may generally be called phasing (lag, i.e. the base that should react in cycle N, but lags to cycle N+1) and prephasing (lead, i.e. the base that should react in Cycle N, but reacts in Cycle N-1), and may be simply understood as crosstalk between adjacent extensions in the same channel. Such a crosstalk is different from the fixed crosstalk between channels, for the former accumulates and becomes stronger as the extension cycle increases. According to embodiments of the present disclosure, phasing/prephasing correction and crosstalk correction both basically involve three steps of "sampling, linear fitting and correction". According to embodiments of the present disclosure, the crosstalk correction may be performed first, and then the phase imbalance crosstalk correction, under which the lowest error rate could be obtained.

For example, FIG. 3 shows a schematic diagram illustrating mixing of base signals with different contents according to an embodiment of the present disclosure. By performing simultaneous sequencing on a reference sample, i.e. a nucleic acid sample with known sequence, to statistically analyze data of multiple base signal mixing, the obtained results could be used as references, and sequencing signals during sequencing could be classified by comparing sequencing results with the references.

S12: Acquiring base types at the assigned position based on a difference between the sequencing signals, thereby achieving sequencing at the first position.

This step includes: classifying the sequencing signal at the first position into a sequence result of corresponding nucleic acid template, according to an intensity difference between the sequencing signals in each channel. Conventionally, for a given sequencing, only the sequencing signal in the channel with the strongest intensity will be selected to determine the result of the nucleic acid template. For example, when there is significant signal difference between the sequencing signals obtained by channels and the strongest sequencing signal has obvious dominant advantage, the base type corresponding to the channel with the strongest sequencing signal will be determined as the base introduced onto the nucleic acid template in the current cycle of sequencing, and the sequencing signals of other channels will be discarded. In some cases, in order to avoid the impact of interference among sequencing signals in multi-channel on base recognition, all data from this signal point is even discarded. This will cause data loss at some sites and reduce sequencing throughput.

In contrast, the inventors of the present disclosure propose that, based on the intensity difference of the sequencing signals of respective channels, the sequencing signals could be classified into different nucleic acid templates, so as to obtain sequencing results of different sequencing templates, thereby improving the sequencing speed and reducing sequencing data waste. Even through single sequencing, sequencing results of multiple nucleic acid templates can be obtained. In other words, through the method according to embodiments of the present disclosure, sequencing signals generated by multiple sequencing templates can be simultaneously acquired at one data position, and these sequencing signals could be classified into different sequencing templates according to the intensity difference among the sequencing signals, so that sequencing results of multiple sequencing templates can be obtained through sequencing at the same signal position.

According to embodiments of the present disclosure, since different nucleic acid templates generate sequencing signals with different intensities in simultaneous sequencing, for determining respective sequencing results of the nucleic acid templates, sequencing signal data at the first position may be acquired to determine sequencing results of multiple different nucleic acid templates based on the intensity difference among the sequencing signals.

In some embodiments, classifying the sequencing signal at the first position into a sequence result of corresponding nucleic acid template, according to an intensity difference between the sequencing signals in each channel includes the following steps:

i. determining, for each cycle of sequencing, the number and intensity of the sequencing signals in respective channels for the first position.

This step includes making a correspondence for the signals generated by base extension according to the location of the position which may also be regarded as the coordinates of the first position. For one first position, the sequencing signals of channels at this position in each cycle of base extension will include at least two sequencing signals with different intensities, that is, among channels the sequencing signals obtained in, at least two channels at the first position present sequencing signals, and the sequencing signal intensities of the two channels are different, or during one cycle of sequencing, in the case of one channel collects signals of two or more base or nucleotide types, this channel detects sequencing signals with different intensities during at least two signal detections. In one embodiment, the sequencing signal is an optical signal, for example, such as a fluorescent signal.

It should be understood that, in this embodiment, it is required to, before sequencing, determine the type and content ranking of each nucleic acid template at the first position by the difference in the number and intensity of sequencing signals presented in base extension. Such a step includes determining the number of the nucleic acid templates and the intensity of each nucleic acid template based on the number of channels having sequencing signals and the intensity of sequencing signals within the channels obtained from the first position in at least one cycle of base extension.

Brief description will be made herein by taking the first position containing two nucleic acid templates as an example. For example, in images generated by multiple cycles of base extension for which four types of nucleotides are simultaneously introduced into the reaction system, and signals of various nucleotides are collected through four channels separately. If, for a fixed position, there are always two channels presenting sequencing signals in the sequencing signals obtained by the four channels, and signal intensity of the two sequencing signals is at a relatively constant ratio, such as 7:3, then it is determined that the copy number of the two nucleic acid templates in this position is about 7:3, according to the signal intensity. In other examples, it may also sequentially introduce four types of nucleotides into the reaction system to perform base extension and corresponding reaction signal collection separately, so as to collect signals of various nucleotides through four channels at one time. If the single channel always detects signals twice out of four signal detections, and signal intensity of the sequencing signals twice obtained is at a relatively constant ratio, such as 7:3, then it is determined that the copy number of the two nucleic acid templates in this position is about 7:3, according to the signal intensity. Certainly, the sequencing method is not limited to this, and may also adopt a method such as the pairwise combination or tri-combination as described above. For convenience of description, a nucleic acid template with high content may be named as the first nucleic acid template, and a nucleic acid template with low content may be named as the second nucleic acid template.

It should be understood that the intensity or signal intensity referred to in the step may be an original intensity of the fluorescence image or a corrected signal intensity. As previously described, the correction may be crosstalk correction and/or phase imbalance correction.

ii. Sorting, in ascending or descending order, the sequencing signals in each cycle of sequencing according to the intensity of the sequencing signals.

In one cycle of sequencing, the n nucleic acid templates at the first position may present sequencing signals in different channels, and when the detection channels are less than four, it may collect different sequencing signals multiple times in one channel. Due to the n nucleic acid templates having different copy numbers at one position, the intensity of these sequencing signals will be different. Sequencing signals may be sorted according to their different intensity in

channels presenting sequencing signals. For example, taking four channels as an example, at the first position containing two nucleic acid templates, if the nucleotides introduced onto the two nucleic acid templates in a cycle of sequencing are different, two different channels in this cycle of sequencing will present bright spots for the first position. Because the copy numbers of the two nucleic acid templates are different, the intensity of the sequencing signals in the two channels will be different.

Specifically, the step ii includes: sorting, according to the intensity of the sequencing signals, the sequencing signals of each channel in each cycle of sequencing based on the intensity of each sequencing signal at the location corresponding to the first position.

Taking fluorescence intensity as an example, at the first position containing two nucleic acid templates where the copy number of the first nucleic acid template to that of the second nucleic acid template is about 7:3, both the A base channel and T base channel show fluorescence signals in the fluorescence image collected by one cycle of sequencing, and the fluorescence signal intensity of the two channels is of 7:3. At this case, the sequencing signals obtained from the two channels are sorted according to the order of fluorescence signal intensity from high to low or from low to high. For example, according to the order of the fluorescence signal intensity from high to low, the sequencing signals of channels are ranked as a first sequencing signal and a second sequencing signal.

It should be understood that, in this step, it is able to know the base type corresponding to a high and low signal intensity by sorting sequencing signals in each cycle of sequencing. Continuing with the above example, at the first position containing two nucleic acid templates where the copy number of the first nucleic acid template to that of the second nucleic acid template is about 7:3, both the A base channel and T base channel show fluorescence signals in the fluorescence image collected by one cycle of sequencing, and the fluorescence signal intensity of the two channels is of 7:3. At this case, the first sequencing signal corresponds to the base type of A, and, correspondingly, the base introduced onto the first nucleic acid template in this cycle of sequencing is A; while the second sequencing signal corresponds to the base type of T, and, correspondingly, the base introduced onto the second nucleic acid template in this cycle of sequencing is T.

It should be understood that in some embodiments, there may be cases where n nucleic acid templates are introduced with the same nucleotide type in one cycle of sequencing, in which case, this position will exhibit a sequencing signal in one channel, or this position will exhibit a sequencing signal in one channel that is much higher than that of other channels, so that with such a signal intensity, it can be determined that this cycle of sequencing introduces only one type of nucleotide or nucleotide analog at this position.

iii. Determining the sequencing result for each nucleic acid template by concatenating the sequencing signal from each cycle of sequencing based on a sorting result.

In one embodiment, determining the sequencing result for each nucleic acid template by concatenating the sequencing signal from each cycle of sequencing based on a sorting result includes:

determining the sequencing signals with the same sorting rank in each cycle of sequencing based on the sorting result;
determining the sequencing signals with the same sorting rank as the sequencing signals from one nucleic acid template; and
concatenating the sequencing signals from one nucleic acid template in each cycle of sequencing, and obtaining the sequencing result of the nucleic acid template based on the concatenated sequencing signal.

Therefore, after sequencing signals obtained from channels in each cycle of sequencing are sorted, a sequence of the nucleic acid template with the highest copy number or the lowest copy number at the first position can be obtained by sequentially concatenating base types corresponding to the sequencing signals ranked first in each cycle of sequencing. By analogy, sequences of the nucleic acid templates with reduced or increased copy number can be obtained in turn according to the sequence signal from strong to weak or from weak to strong.

Illustratively, a pattern chip is taken as an example to illustrate an identification to two sequences of two different nucleic acid templates located in two nucleic acid templates contained at a first position. In principle, there may be dimolecular signal points formed by amplification of two DNA sequences in a nanowell on the pattern chip. Due to the difference in amplification order and amplification efficiency, the amplification quantity ratio of the two DNA sequences is 7:3. Fluorescence images of base channels are collected in each cycle of sequencing, and four gray values may be extracted from the images. Further, fluorescence intensity in the image may be performed with crosstalk correction and/or phase imbalance correction. If the base signals of the two nucleic acid templates are different, as A and C, in one cycle of sequencing, the intensity ratio of the four bases in the image will be 7:3:0:0 (A:C:G:T, assuming that the total ratio of the signals of the four channels is 10), thus recognizing base A and the base C. In the next cycle of sequencing, if the bases of the two nucleic acid templates are both T, and the intensity ratio of the four bases in the image is 0:0:0:10, it is determined that the two bases introduced in this cycle of sequencing are both T. In each cycle of sequencing, a base with the maximum intensity and a base with the second maximum intensity in the image are identified and ranked according to light to dark, and bases with the maximum intensity are concatenated one by one, and bases with the second intensity are concatenated one by one, thus to obtain the sequences of two nucleic acid templates, thereby realizing output of two

sequences from one nano well, namely realizing double molecule recognition.

The second implementation case: there are a plurality of identical nucleic acid templates at one position, the plurality of nucleic acid template as identical having multiple different target nucleic acid sequences, to perform simultaneous sequencing on the multiple target nucleic acid sequences.

In the second implementation case, the surface of the solid support includes a class of positions which includes a plurality of nucleic acid templates. Unlike the first implementation case, the plurality of nucleic acid templates in this position are copies of an identical nucleic acid molecule, that is, the nucleic acid templates in this position are identical. Further, each nucleic acid template among these nucleic acid templates includes at least one target nucleic acid sequence, and at least part of these nucleic acid templates each includes m different target nucleic acid sequences. In such positions, at least two of the different target nucleic acid sequences generate different sequencing signals in at least one cycle of sequencing. For ease of description and distinction, such a position is referred to simply as "a second position". It should be understood that the second position contains two or more target nucleic acid sequences, but this does not mean that each second position has the same number of target nucleic acid sequences. Illustratively, at some second positions there are two target nucleic acid sequences from one nucleic acid template; at other second positions there are three target nucleic acid sequences from one nucleic acid template; and at still other second positions there are four or more (e.g., four, five, or more) target nucleic acid sequences from one nucleic acid template. Certainly, the case that all the second positions contain the same number of target nucleic acid sequences is also within the scope of the present disclosure, and could be also realized with the determination of the base or nucleotide type and sequencing on the nucleic acid sequence through the sequencing method provided by the present disclosure.

In some embodiments, the second position includes m different target nucleic acid sequences. In some embodiments, at one second position, each nucleic acid template includes m distinct primer hybridization sites, the m different target nucleic acid sequences are respectively ligated to 3' ends of the m distinct primer hybridization sites, thus to generate m different target nucleic acid sequences on one nucleic acid template. Each primer hybridization site is used to extend the target nucleic acid sequence that is ligated to its 3' end.

According to embodiments of the present disclosure, the nucleic acid template at the second position is hybridized to m primers at m distinct primer hybridization sites, and at the same second position, the amounts of the m hybridized primers differ. That is, in a plurality of identical target nucleic acid sequences at the second position, there is randomness for primers hybridized with distinct primer hybridization sites, such that target nucleic acid sequences capable of being sequenced have different content at the second position. Accordingly, in one cycle of sequencing, the sequencing signals obtained in dominant base signal channels for each target nucleic acid sequence can be distinguished, thus realizing assignment of the sequencing signal to each base template.

It should be understood that at one second position, the greater the number difference of the m primers corresponding to the m primer hybridization sites, the more conducive to distinguish multiple sequencing signals generated in the sequencing. In some embodiments, if the primer hybridization site with the highest number of primers hybridized thereto is 100%, the number difference among the primers hybridized to m primer hybridization sites may be between 5% and 95%, which is beneficial to render the number of the target nucleic acid sequences capable of being sequenced differential based on the differentiated number of primers. In some embodiments, if the primer hybridization site with the highest number of primers hybridized thereto is 100%, the number difference among the primers hybridized to m primer hybridization sites may be between 20% and 80%. Illustratively, when m=2, a ratio of primers hybridized to the two primer hybridization sites may be 10:8, 10:7, 10:6, 10:5, 10:4, 10:3, 10:2, etc. It should be understood that it is unable to count the numbers of primers hybridized to the primer hybridization sites at the second position, the ratio herein may be achieved by controlling, for example, the concentration of primers introduced in the primers hybridizing to respective primer hybridization site in the nucleic acid template, such as controlling a relative ratio between extendable forward primers and non-extendable blocking molecules hybridized to one sequencing primer binding site. In addition, the ratio above is only used to reflect a trend, and the actual ratio may fluctuate to a certain extent on this basis. In theory, it is feasible as long as the difference in the amount of the two is sufficient to recognize the difference in sequencing signals generated by the sequencing.

In embodiments of the present disclosure, m is an integer greater than or equal to 2, i.e., there are two or more target nucleic acid sequences at the same second position. It should be understood that the greater the number of target nucleic acid sequences at the same second position, the more complex the corresponding sequencing signals generated during the sequencing, the more complicated algorithm for analyzing the sequencing data corresponding to the sequencing signals and realizing the assignment of each sequencing signal. In some embodiments, m is selected from 2 to 4, illustratively m is 2, 3, or 4. In this case, the number of target nucleic acid sequences from one nucleic acid template at one second position is controlled within 4. A relative content of each target nucleic acid sequence at the second position is judged according to the signal collected by each channel in one or more cycles of sequencing and the intensity of the signal in each channel. In an embodiment, different target nucleic acid sequences at one second position generate different sequencing signals in at least one cycle of sequencing, and in this case, the relative content of each target nucleic acid sequence at the second position could be determined according to the sequencing signal intensity generated in this cycle

of sequencing. In another embodiment, different target nucleic acid sequences at one second position generate the same portion of sequencing signals in one cycle of sequencing, and in this case, the relative contents of target nucleic acid sequences with different sequencing signals at the second position could be determined through one cycle of sequencing. The remaining target nucleic acid sequences whose relative content has not been determined will generate different sequencing signals during other cycles of sequencing, and in this case, their relative content can be determined by identifying the intensity of sequencing signals based on the same method. With two or more cycles of such sequencing, the content of each target nucleic acid sequence at one second position could be determined. On this basis, the base or nucleotide type introduced onto each target nucleic acid sequence in the current cycle of sequencing could be determined based on the relative content of each target nucleic acid sequence at this second position and the sequencing signal intensity generated in each cycle of sequencing. Even if two target nucleic acid sequences produce the same sequencing signals, the sequencing signal may also be assigned based on the fixed content of each nucleic acid template. That is, for the second position containing multiple target nucleic acid sequences on identical nucleic acid templates, the base or nucleotide type of each cycle of sequencing could be called according to signal characteristics, and finally, according to sequencing signals such as collected fluorescent images obtained by multiple cycles of sequencing, sequences of the multiple target nucleic acid sequences could be identified from one second position.

Illustratively, a second position containing two different target nucleic acid sequences is taken as an example to illustrate an identification to two sequences of two different target nucleic acid sequences located at one nucleic acid template. Before base recognition, library molecules may be designed such that the nucleic acid template has two distinct primer hybridization sites. At this point, there is generated a dimolecular signal point at this second position in each cycle of sequencing. By controlling the effectively available rate of primer hybridization sites, for example, one of the primer hybridization sites is blocked with blocking molecules on a part thereof thus blocking extension at the 3' end, such that the two target nucleic acid sequences as templates have different content. At this point, in each cycle of sequencing there is generated the dimolecular signal point at this second position. The two target nucleic acid sequences, in each cycle of sequencing, also present a different but relatively constant ratio in signal intensity generated in a certain two base channels. Based on the characteristics of the sequencing signal obtained by each cycle of base extension, the base or nucleotide type introduced onto each of the two target nucleic acid sequences in each cycle of base extension could be known.

According to embodiments of the present disclosure, intensity differences among the sequencing signals may be realized by controlling the amount of primers capable of performing extension in the sequencing. Specifically, according to embodiments of the present disclosure, at least a portion of the target nucleic acid sequences may be blocked with at least one blocking molecule, such that multiple different target nucleic acid sequences generate sequencing signals with different intensities.

In some embodiments, a portion of primer hybridization sites in the nucleic acid template at the second position is blocked, resulting in different nucleic acid templates at one second position with different amounts, where the target nucleic acid sequence with blocked hybridization sites loses its function as a sequencing nucleic acid template. Illustratively, by introducing non-extendable blocking molecules capable of hybridizing to the primer hybridization sites, different nucleic acid templates at one second position thus have different amounts. In an embodiment, at least m-1 distinct primer hybridization sites in the nucleic acid template at the second position bind non-extendable blocking molecules.

In some embodiments, the blocking molecule is a nucleic acid primer whose 3' end is non-extendable. For example, according to embodiments of the present disclosure, at least one blocking molecule has substantially the same sequence as a sequencing primer corresponding to at least one primer hybridization site in the target nucleic acid sequence, and the blocking molecule has a non-extendable 3' end. Accordingly, by controlling the amount of blocking molecules, it is realized to block the sequencing primer sites recognized by a predetermined proportion of the target nucleic acid sequences, for example, half of the target nucleic acid sequences of the nucleic acid template, so as to differentiate the intensitie of the sequencing signals generated at the second position.

In an embodiment, the blocking molecule includes a nucleotide at the 3' end. With modifications to the structure of the nucleotide at the 3' end, the nucleotide at the 3' end loses the function of bonding to a phosphate group at its 3' end and thus loses the function of extension. Illustratively, in the 3' end nucleotide as the blocking molecule, 3-OH of the ribose group or deoxyribose group is replaced with a group or atom that is disable to react with a phosphate group, such as a hydrogen atom, -NH2, etc., but is not limited thereto. Theoretically, any situation capable of blocking reaction between the nucleotide at 3' end and the phosphate of the next nucleotide may be used in embodiments of the present disclosure.

In an embodiment of the second implementation case, the second position containing multiple target nucleic acid sequences at different proportions may be obtained by:

amplifying hybrids obtained by hybridizing the nucleic acid templates onto the surface of the solid support; and introducing onto the solid support the m primers respectively hybridized to the m primer hybridization sites and m-1 blocking molecules respectively hybridized to the m-1 primer hybridization sites, such that the nucleic acid templates at one or more positions are hybridized to the primers at distinct primer hybridization sites with different amounts, and

designating each of the one or more position as the second position.

In this embodiment, due to the introduction of blocking molecules that are competitive for binding primer hybridization sites thus blocking the extension of partial target nucleic acid sequences, the number of primers hybridized to m primer hybridization sites in the nucleic acid template at the same second position will be different, that is, the number of m target nucleic acid sequences that is extendable in the nucleic acid template is different.

In some embodiments, for any primer hybridization site of the m primer hybridization sites, the sum of the introduced primer and blocking molecule number is constant, and by controlling a ratio of the blocking molecule to the total number of primers and blocking molecules, the nucleic acid template at the second position has different number of primers hybridized at distinct primer hybridization sites therein.

Illustratively, there are three primer hybridization sites set on the nucleic acid template, and are named as primer hybridization site No. 1, primer hybridization site No. 2 and primer hybridization site No. 3 from 5' end to 3' end respectively. Primers corresponding to the primer hybridization site No. 1, primer hybridization site No. 2 and primer hybridization site No. 3 are primer No. 1, primer No. 2 and primer No. 3 respectively, and blocking molecules corresponding to the three primer hybridization sites are blocking molecule No. 1, blocking molecule No. 2 and blocking molecule No. 3 respectively. For hybridization of the primers to the primer hybridization site, 20% blocking molecule No. 2 and 50% blocking molecule No. 3 are added based on 100% of the total amount of primers and blocking molecules introduced to each primer hybridization site, with 0% blocking molecule No. 1 introduced, thereby making it different in primer amounts hybridized to the nucleic acid template at the one position.

In another embodiment of the second implementation case, the second position may be obtained by:

amplifying hybrids obtained by hybridizing the nucleic acid templates onto the surface of the solid support; and introducing onto the solid support the m primers with different concentrations respectively hybridized to the m primer hybridization sites, such that the nucleic acid templates at one or more positions are hybridized to the primers at distinct primer hybridization sites with different amounts, and designating each of the one or more positions as the second position.

In this embodiment, due to m primers having different concentrations, the number of primers hybridized to m primer hybridization sites in the nucleic acid template at the same second position will be different, that is, the copy number of m target nucleic acid sequences in the nucleic acid template is different.

Illustratively, there are three primer hybridization sites set on the nucleic acid template, and are named as primer hybridization site No. 1, primer hybridization site No. 2 and primer hybridization site No. 3 from 5' end to 3' end respectively. Primers corresponding to the primer hybridization site No. 1, primer hybridization site No. 2 and primer hybridization site No. 3 are primer No. 1, primer No. 2 and primer No. 3 respectively. For hybridization of the primers to the primer hybridization site, three primers are introduced with a content ratio of 10:7:5 based on their concentrations, along with control of hybridization time, thereby making it different in primer amounts hybridized to the nucleic acid template at the one position.

In still another embodiment of the second implementation case, the second position may be obtained by:

amplifying hybrids obtained by hybridizing the nucleic acid templates onto the surface of the solid support; and introducing onto the solid support the m primers with different lengths respectively hybridized to the m primer hybridization sites, such that the nucleic acid templates at one or more positions are hybridized to the primers at distinct primer hybridization sites with different amounts, and designating each of the one or more positions as the second position.

In this embodiment, the concentration of m primers is different when hybridizing to primer hybridization sites. In general, primers with high concentrations hybridize faster, while primers with low concentrations hybridize slower. Accordingly, by controlling the concentration of m primers, the number of primers hybridized to m primer hybridization sites in the nucleic acid template at the same second position will be different, that is, the copy number of m target nucleic acid sequences in the nucleic acid template is different.

Illustratively, there are three primer hybridization sites set on the nucleic acid template, and are named as primer hybridization site No. 1, primer hybridization site No. 2 and primer hybridization site No. 3 from 5' end to 3' end respectively. Primers corresponding to the primer hybridization site No. 1, primer hybridization site No. 2 and primer hybridization site No. 3 are primer No. 1, primer No. 2 and primer No. 3 respectively. For hybridization of the primers to the primer hybridization site, three primers are introduced with a content ratio of 10:7:5 based on their concentrations, along with control of hybridization time, thereby making it different in primer amounts hybridized to the nucleic acid template at the one position.

In yet another embodiment of the third implementation case, the second position may be obtained by:

amplifying hybrids obtained by hybridizing the nucleic acid templates onto the surface of the solid support, so as to immobilize a plurality of nucleic acid templates at one position, the nucleic acid template containing m primer hybridization sites; and

introducing onto the solid support the m primers with different lengths respectively corresponding to the m primer hybridization sites, such that the nucleic acid templates at one or more positions are hybridized to the primers at distinct primer hybridization sites with different amounts, and designating each of the one or more positions as the second position.

In this embodiment, m primers are of different lengths. The different length of primer referred to herein refers to the difference in the base numbers composed of primer nucleic acid fragments. In general, primers with long lengths have stronger stability after hybridization and are more likely to remain in the nucleic acid template. Accordingly, when the length difference is significant, there will be differences in the number of primers with different lengths finally bound to the second position. Thus, by controlling lengths of m primers, the number of primers hybridized to m primer hybridization sites in the nucleic acid template at the same second position will be different, that is, the number of m target nucleic acid sequences that is extendable in the nucleic acid template is different.

In some embodiments, each nucleic acid template at the second position includes:

a first primer hybridization site;

a first target molecule, ligated with a 3' end of the first primer hybridization site;

a second primer hybridization site, located on the side distal to the first target molecule; and

a second target molecule, ligated with a 3' end of the second primer hybridization site.

In this case, the first target molecule and the second target molecule are two target nucleic acid sequences.

In an embodiment, the first target molecule includes a first barcode sequence and a first insert fragment, the second target molecule includes a second barcode sequence and a second insert fragment, and the first barcode sequence and the second target molecule are of different nucleic acid sequences.

Referring to FIG. 4, a schematic diagram showing a structure of a nucleic acid template according to an embodiment of the present disclosure, a sequencing library molecule includes i. a first amplification primer and a target molecule 1; and ii. a second amplification primer and a target molecule 2, where the target molecule 1 may be complementary to at least part of the target molecule 2 to form a complementary sequence. Further, there are a hybridization site of primer 1 at the upstream of the target molecule 1, and a hybridization site of primer 2 at the upstream of the target molecule 2, thereby realizing simultaneous sequencing on the target molecule 1 and the target molecule 2 through synchronous sequencing. According to embodiments of the present disclosure, the target molecule 1 and target molecule 2 may also be provided with the same or different barcodes (sometimes also referred to as "indexes") or UMI sequences, so as to distinguish sample sources or molecules, thereby avoiding barcode alignment errors in multiplex libraries.

In an embodiment, the nucleic acid template at the second position is obtained by:

a. ligating a first adapter including the first primer hybridization site to an end of the first target molecule;

b. ligating, via a second adaptor including the second primer hybridization site, the second target molecule to a product of step a; and

c. amplifying a product of step b.

In an embodiment, the first insert fragment is complementary to the second insert fragment to form a complementary sequence. Correspondingly, the nucleic acid templates at the second position are obtained by:

i. ligating a first adapter and a second adapter respectively to two 5' terminals of a double-strand target molecule of two nucleic acid templates, the first adapter containing the first primer hybridization site;

ii. ligating, via a cyclizing adapter including the second primer hybridization site, the first adapter to a 3' terminal of another target molecule;

iii. subjecting a product of step ii to single-strand transformation, to obtain a single-strand nucleic acid template; and

iv. amplifying the nucleic acid template.

Exemplary sequencing methods of a second embodiment include the following steps.

S21: Detecting sequencing signals at the first position of the solid support during sequencing.

With solid support obtained, multiple sequencing cycles are performed on the solid support, and the sequencing signals, at an assigned position, in each base-signal channel are acquired in sequencing. This step includes: performing multiple sequencing cycles on the nucleic acid templates at the second position, to acquire the sequencing signals in each

channel for the second position.

During sequencing, for each nucleic acid template, each cycle of sequencing extends a nucleotide or analog thereof conjugated with a specific signal group (usually a fluorescent group). By detecting the sequencing signal (usually a fluorescent signal) generated by the specific signal group in a specific channel, the base type or nucleotide extended and thus the sequence of the nucleic acid template can be determined.

According to embodiments of the present disclosure, there may be at least two target nucleic acid sequences at the second position. Illustratively, at least two of different target nucleic acid sequences at the second position generate different sequencing signals in at least one cycle of sequencing.

In embodiments of the present disclosure, in sequencing, acquiring the sequencing signal of each channel obtained at the second position includes obtaining the sequencing signal of each channel in each cycle of sequencing. In some embodiments, the sequencing signal is an optical signal. Illustratively, the sequencing signal is a fluorescent signal generated by a fluorescent group on a nucleotide, and in this case, acquiring the sequencing signal of each channel includes: acquiring fluorescent images of four base channels after a cycle of sequencing, and acquiring a bright spot signal in the fluorescent image of each channel. A bright spot in the fluorescent image corresponds to a signal of a site. Correspondingly, the signal generated at the second position is a multi-signal, and in this case, the corresponding bright spot is a bright spot formed by multiple signals mixed. In some embodiments, acquiring the bright spot signal in the fluorescent image of each channel includes determining fluorescence intensity of each bright spot in the fluorescent image. Specifically, the fluorescent image may be extracted with a gray scale value, and the fluorescence intensity of each bright spot in the fluorescent image may be confirmed after performing a correction to the gray scale image obtained. Exemplary corrections include crosstalk correction and/or phase imbalance correction, including phasing and prephasing correction.

Since a plurality of different signals are generated at the second position containing a plurality of target nucleic acid sequences during each cycle of sequencing and there may be crosstalk among these signals, the correction is performed on sequencing signal data to eliminate interference after obtaining the sequencing signal data of the target nucleic acid sequences, according to an embodiment of the present disclosure. This procedure can further improve the accuracy of sequencing. For example, FIG. 2 shows a schematic diagram illustrating mixing of base signals with different contents according to an embodiment of the present disclosure. By performing simultaneous sequencing on a reference sample, i.e. a nucleic acid sample with known sequence, to statistically analyze data of multiple base signal mixing, the obtained results could be used as references, and sequencing signals during sequencing could be classified by comparing sequencing results with the references.

S12: Acquiring base types at the assigned position based on a difference between the sequencing signals, thereby achieving sequencing at the first position.

This step includes: classifying the sequencing signal at the second position into a sequence result of corresponding target nucleic acid sequence, according to an intensity difference between the sequencing signals in each channel. Conventionally, for a given sequencing, only the sequencing signal in the channel with the strongest intensity will be selected to determine the result of the nucleic acid template. If there is significant signal difference and the strongest sequencing signal has obvious dominant advantage, the base type corresponding to such a strongest channel will be determined as the base introduced onto the target nucleic acid sequence in the current cycle of sequencing, while the sequencing signals of other channels will be discarded, and even all data from this signal point will be discarded. In contrast, the inventors of the present disclosure propose that, with multiple different target nucleic acid sequences provided on a plurality of nucleic acid templates at one position, based on the intensity difference of the sequencing signals of respective channels, the sequencing signals could be classified into different target nucleic acid sequences, so as to obtain sequencing results of different target nucleic acid sequences, thereby improving and doubling the sequencing speed. Even through single sequencing, sequencing results of multiple target nucleic acid sequences can be obtained. In other words, through the method according to embodiments of the present disclosure, sequencing signals generated by multiple target nucleic acid sequences can be simultaneously acquired at one data position, and these sequencing signals could be classified into different target nucleic acid sequences according to the intensity difference among the sequencing signals, so that sequencing results of multiple target nucleic acid sequences can be obtained through sequencing at the same signal position.

According to embodiments of the present disclosure, since different target nucleic acid sequences generate sequencing signals with different intensities in simultaneous sequencing, for determining the sequencing results of the target nucleic acid sequences, sequencing signal data at the second position may be acquired to determine sequencing results of multiple target nucleic acid sequences based on the intensity difference among the sequencing signals.

In some embodiments, classifying the sequencing signal at the second position into a sequence result of corresponding target nucleic acid sequence, according to an intensity difference between the sequencing signals includes:

i. determining a value of the m for the second position and relative signal intensities attributable to each of the m target

nucleic acid sequences, based on a number of the channels having the sequencing signals and an signal intensity in each channel acquired from the second position in at least one cycle of sequencing.

This step includes making a correspondence for the signals generated by sequencing according to the position which may also be regarded as the coordinates of the second position. That is, the sequencing signals generated in each cycle of sequencing are subjected to classification by determining the coordinates the second position corresponds to. For a second position, the sequencing signals of channels at one second position in each cycle of sequencing will include at least two sequencing signals with different intensities, that is, among channels the sequencing signals obtained in, at least two channels at the second position present sequencing signals, and the sequencing signal intensities of the two channels are different, or during one cycle of sequencing, in the case of one channel collects signals of two or more base or nucleotide types, this channel detects sequencing signals with different intensities during at least two signal detections. In one embodiment, the sequencing signal is an optical signal, for example, such as a fluorescent signal.

A value of the m for the second position and relative signal intensities attributable to each of the m target nucleic acid sequences are determined, based on a number of the channels having the sequencing signals and an signal intensity in each channel acquired from the second position.

The copy numbers of the m target nucleic acid sequences at the second position are different, resulting in different sequencing signal intensities generated by the m target nucleic acid sequences at the second position during sequencing. This step includes determining, during the sequencing at the second position, the number of channels that present sequencing signals and number of times for presenting sequencing signals in a channel, to analyze the number of target nucleic acid sequences with the number and the number of times of channels that present sequencing signals, i.e., determining the m value at the second position. According to the sequencing signal intensities obtained at the second position, the relative signal intensities of the m target nucleic acid sequences can be analyzed.

Illustratively, for example, in images generated by multiple cycles of sequencing for which four types of nucleotides are simultaneously introduced into the reaction system, and signals of various nucleotides are collected through four channels separately. If, for a fixed position, there are always two channels presenting sequencing signals in the sequencing signals obtained by the four channels, and signal intensity of the two sequencing signals is at a relatively constant ratio, such as 7:3, then it is determined that there are two target nucleic acid sequences at this second position, and a ratio of the number of the two target nucleic acid sequences in this position is about 7:3, according to the signal intensity. In other examples, it may also sequentially introduce four types of nucleotides into the reaction system to perform base extension and corresponding reaction signal collection separately, so as to collect signals of various nucleotides through four channels at one time. If the single channel always detects signals twice out of four signal detections, and signal intensity of the sequencing signals twice obtained is at a relatively constant ratio, such as 7:3, then it is determined that the copy number of the two nucleic acid templates in this position is about 7:3, according to the signal intensity. Certainly, the sequencing method is not limited to this, and may also adopt a method such as the pairwise combination or tri-combination as described above. For convenience of description, a target nucleic acid sequence with high content may be named as a first target nucleic acid sequence, and a target nucleic acid sequence with low content may be named as a second target nucleic acid sequence.

It should be understood that the signal intensity referred to in the step may be an original intensity of the fluorescence image or a corrected signal intensity. As previously described, the correction may be crosstalk correction and/or phase imbalance correction.

ii. Sorting the sequencing signals in each cycle of sequencing according to the relative signal intensities in channels for the second position in each cycle of sequencing.

In one cycle of sequencing, the m target nucleic acid sequences at the second position may present sequencing signals in different channels, and when the detection channels are less than four, it may collect different sequencing signals multiple times in one channel. Due to target nucleic acid sequences of the m nucleic acid sequences having different numbers at one position, the intensity of these sequencing signals will be different. Sequencing signals may be sorted according to their different intensity in channels presenting sequencing signals. For example, taking four channels as an example, at the second position containing two target nucleic acid sequences, if the nucleotides introduced onto the two target nucleic acid sequences in a cycle of sequencing are different, two different channels in this cycle of sequencing will present bright spots for the second position. Because the numbers of extendable target nucleic acid sequences are different in the two nucleic acid sequences, the intensity of the sequencing signals in the two channels will be different.

Specifically, the step ii includes: sorting, according to the intensity of the sequencing signals, the sequencing signals in each cycle of sequencing based on the intensity of sequencing signals at the second position.

Taking fluorescence intensity as an example, at the second position containing two target nucleic acid sequences where the number of the first target nucleic acid sequence to that of the second target nucleic acid sequence is about 7:3, both the A base channel and T base channel show fluorescence signals in the fluorescence image collected by one cycle of sequencing, and the fluorescence signal intensity of the two channels is of 7:3. At this case, the two sequencing signals are sorted according to the order of fluorescence signal intensity from high to low or from low to high. For example, according to

the order of the fluorescence signal intensity from high to low, the sequencing signals are ranked as a first sequencing signal and a second sequencing signal.

It should be understood that, in this step, it is able to know the base type corresponding to a high and low signal intensity by sorting sequencing signals in each cycle of sequencing. Continuing with the above example, at the first position containing two nucleic acid templates where the copy number of the first nucleic acid template to that of the second nucleic acid template is about 7:3, both the A base channel and T base channel show fluorescence signals in the fluorescence image collected by one cycle of sequencing, and the fluorescence signal intensity of the two channels is of 7:3. At this case, the first sequencing signal corresponds to the base type of A, and, correspondingly, the base introduced onto the first nucleic acid template in this cycle of sequencing is A; while the second sequencing signal corresponds to the base type of T, and, correspondingly, the base introduced onto the second nucleic acid template in this cycle of sequencing is T.

It should be understood that in some embodiments, there may be cases where m target nucleic acid sequences are introduced with the same nucleotide type in one cycle of sequencing, in which case, this position will exhibit a sequencing signal in one channel, or this position will exhibit a sequencing signal in one channel that is much higher than that of other channels, so that with such a signal intensity, it can be determined that this cycle of sequencing introduces only one type of nucleotide at this position.

iii. Determining the sequencing result for each target nucleic acid sequence by concatenating the sequencing signal from each cycle of sequencing based on a sorting result.

In an embodiment, determining the sequencing result for each target nucleic acid sequence by concatenating the sequencing signal from each cycle of sequencing based on a sorting result includes:

determining the sequencing signals with the same sorting rank in each cycle of sequencing based on the sorting result;
determining the sequencing signals with the same sorting rank as the sequencing signals from one target nucleic acid sequence; and
concatenating the sequencing signal from one target nucleic acid sequence in each cycle of sequencing, and obtaining the sequencing result of the target nucleic acid sequence based on the concatenated sequencing signal.

Therefore, after sequencing signals obtained from channels in each cycle of sequencing are sorted, a sequence of the target nucleic acid sequence with the highest content or the lowest content at the second position can be obtained by sequentially concatenating base types corresponding to the sequencing signals ranked first in each cycle of sequencing. By analogy, sequences of the target nucleic acid sequences with reduced or increased content can be obtained in turn according to the sequence signal from strong to weak or from weak to strong.

In embodiments of the present disclosure, the solid support includes on its surface a third position in which the nucleic acid templates are identical and each nucleic acid template has one identical primer hybridization site. That is, there is one target nucleic acid sequence on the nucleic acid template and such a target nucleic acid sequence is the entire sequence of the nucleic acid template.

For positions on the surface of the solid support, there may be a combination of the first position and the third position, a combination of the second position and the third position, or a combination of the first positions, the second position, and the third position. In some cases, positions on the surface of the solid support may further all be the first positions, or a combination of the first position and the second position.

The inventor of the present disclosure verifies through experiments the technical concept that sequencing signals could be effectively distinguished with intensity differences by performing synchronous sequencing on different target nucleic acid sequences, thereby greatly improving the sequencing speed. The method is especially suitable for sequencing libraries carrying multiple barcodes.

Specifically, during NGS sequencing on multiple samples, each nucleic acid molecule will be introduced with a specific sequence uniquely corresponding to the sample from which the nucleic acid molecule comes in preparing a library for the nucleic acid molecule, allowing the library containing multiple samples to be mixed into a reaction system for sequencing. After sequencing on the nucleic acid molecule, sequencing signals or sequencing data obtained from the sequencing signals may be assigned to corresponding samples according to the specific sequence to which the nucleic acid molecule is ligated, thereby obtaining sequencing data for each sample. The specific sequence referred to herein is also referred to as an index or barcode.

There may be correspondence errors during barcode assignment among multiplex libraries, which is also known as index hopping, a common problem in index sequencing. A common method to solve index hopping is dual-indexing sequencing, that is, by introducing indexes into adapters at both ends of the nucleic acid molecule to be sequenced, it is strictly required that only when the indexes at ends are both matched, can the library indexes be confirmed. However, existing dual-indexing sequencing requires multiple cycles of sequencing. As shown in FIG. 5, both ends of the nucleic acid molecule have unique dual indexes (UDIs), Index i5 and Index i7, respectively. Currently, a sequencing process for such a dual-indexed nucleic acid molecule usually includes: sequencing the nucleic acid molecule (i.e., Read1 sequencing in FIG. 4), then sequencing one of the indexes, Index i7, and sequencing the other index, Index i5, thereby realizing the

sequencing of multiple nucleic acid samples on the surface of the solid support through the three-step sequencing processes.

In contrast, in the sequencing method provided in embodiments of the present disclosure, the two indexes could be sequenced simultaneously, for example, in SBS (Sequencing by Synthesis) sequencing cycles, the two indexes could be sequenced simultaneously with a primer combination, thereby greatly saving time cost for index sequencing.

In an embodiment, there is provided a sequencing method as shown in FIG. 6 and FIG. 7, including:

E10: preparing a sequencing library.

In this step, the sequencing library includes a plurality of nucleic acid molecules, each nucleic acid molecule containing: a first barcode, including a first primer hybridization site and a first barcode sequence ligated with a 3' end of the first primer hybridization site;

a second barcode, including a second primer hybridization site and a second barcode sequence ligated with a 3' end of the second primer hybridization site;

a target molecule, ligated between the first barcode and the second barcode, including: a third primer hybridization site and an insert fragment ligated with a 3' end of the third primer hybridization site, the target molecule is ligated with a 3' end of the first barcode via the third primer hybridization site, and the target molecule is ligated with a 5' end of the second barcode via the insert fragment.

In this case, the library prepared is a dual-indexing nucleic acid molecule library. In some embodiments, the first barcode is complementary to the second barcode.

E20: Amplifying the nucleic acid templates of the sequencing library after immobilized onto a surface of the solid support, to form a position with a nucleic acid molecule cluster.

This step includes unzipping the double-stranded nucleic acids into single-stranded nucleic acid molecules, and then immobilizing the single-stranded nucleic acid molecules onto the surface of solid support. In some embodiments, probes are immobilized on the surface of the solid support, by which the nucleic acid molecules in the sequencing library are immobilized on the surface of the solid support. The immobilized nucleic acid molecules are amplified to form positions on the surface of the solid support at which nucleic acid molecule clusters are immobilized. An exemplary solid support is a pattern chip, and probes are immobilized in nanowells of the pattern chip. Nucleic acid molecules in the sequencing library are attached to the probes, thereby immobilizating on the surface of solid support. By amplification, the nucleic acid molecules each generates a plurality of copies in the nanowell, forming nucleic acid molecule clusters. In this case, a nanowell is equivalent to a position.

E30: Introducing a first primer, a blocking molecule and a second primer to hybridize with the nucleic acid molecule of the nucleic acid molecule cluster, where the first primer and the blocking molecule are hybridized to the first primer hybridization site, the second primer is hybridized to the second primer hybridization site, and the blocking molecule is of a non-extendable 3' end.

This step includes introducing a primer mix such that two primer hybridization sites (i.e. the first primer hybridization site and the second primer hybridization site) in the first barcode and the second barcode are hybridized with different primers, respectively. Specifically, the first primer, the blocking molecule and the second primer are added to hybridize with the nucleic acid template, where the first primer and the blocking molecule are hybridized to the first primer hybridization site, while the second primer is hybridized to the second primer hybridization site, and the blocking molecule has an inextensible 3' end. At this time, the first primer and the blocking molecule competitively are hybridized to the first primer hybridization site of the first barcode, so that one part of the nucleic acid templates is hybridized with the first primers at their first barcodes, while the other part of the nucleic acid templates is hybridized with the blocking molecules at their first barcodes. The second primer is hybridized to the second primer hybridization site of the second barcode such that the second barcodes of the nucleic acid templates are hybridized only to the second primer. It should be understood that the concentration of each primer added is higher than a concentration required for each nucleic acid template to be hybridized. The nucleic acid templates thus formed have a lower content of the hybridized first primer than that of the hybridized second primer, rendering the content of the first barcodes extendable by the first primers differential to that of the second barcodes extendable by the second primers.

In some embodiments, the blocking molecule is used in an amount of 20% to 80% of the amount of the first primer. It should be understood that in embodiments of the present disclosure, the content referred to, such as primer content, may be a molar concentration, molar percentage content, or even quantity, and the parameters expressing the content may be converted to each other as long as a standard of content is unified. Illustratively, the molar content ratio of the blocking molecule to the first primer is 10:8, 10:7, 10:6, 10:5, 10:4, 10:3, 10:2, etc.

In an embodiment, as shown in FIG. 5, before introducing the first primer, the blocking molecule and the second primer to hybridize with the nucleic acid molecule, the method further includes: introducing a third primer to hybridize with the nucleic acid molecule to sequence the insert fragment, where the third primer is hybridized to the third primer hybridization site. At this point, the nucleic acid sequence of the insert fragment is determined before synchronous

sequencing of the double-indexing sequence.

E40: Performing multiple sequencing cycles on the first barcode and the second barcode simultaneously, to acquire sequencing signals in each channel in each cycle of sequencing.

Referring to FIG. 7, multiple cycles of sequencing are simultaneously performed on the first barcode and the second barcode, with the first primer and the second primer as starting points, respectively. In this process, performing multiple cycles of sequencing simultaneously means that in each cycle of sequencing, there is one base or nucleotide extended into a nucleic acid chain where the first primer is located, and, simultaneously, there is one base or nucleotide extended into a nucleic acid chain where the second primer is located.

Specifically, during sequencing, for each of the two barcodes including the first barcode and the second barcode, each cycle of sequencing extends a nucleotide or analog thereof conjugated with a specific signal group (usually a fluorescent group). By detecting the sequencing signal (usually a fluorescent signal) generated by the specific signal group in a specific channel, the base type or nucleotide extended into the sequencing and thus the sequence of the two barcodes including the first barcode and the second barcode can be determined.

In this step, in sequencing, acquiring the sequencing signal of each channel includes obtaining the sequencing signal of each channel in each cycle of sequencing. In some embodiments, the sequencing signal is an optical signal. Illustratively, the sequencing signal is a fluorescent signal generated by a fluorescent group on a nucleotide, and in this case, acquiring the sequencing signal of each channel includes: collecting a fluorescent image of each base channel after a cycle of sequencing, and acquiring a bright spot signal in the fluorescent image of each channel. For a dual-indexing sequencing, there are two signals at one position, i.e., forming a double-signal position. It should be understood that when the channel is with four channels, fluorescent images of the four channels may be acquired at one time; and when the channel is less than four channels, at least one channel needs to be acquired for multiple times to obtain fluorescent images of four bases. Illustratively, when the channel is a dual channel, each channel collects signals of two types of bases, or one channel collects one type of base, and the other channel collects signals of the remaining three types of bases for three times respectively; when the channel is a single channel, the channel performs signal collection four times individually to obtain signals of four types of bases. Certainly, the channel is not limited thereto.

In some embodiments, acquiring the bright spot signal in the fluorescent image of each channel includes determining fluorescence intensity of each bright spot in the fluorescent image. Specifically, the fluorescent image may be extracted with a gray scale value, and the fluorescence intensity of each bright spot in the fluorescent image may be confirmed after performing a correction to the gray scale image obtained. Exemplary corrections include crosstalk correction and/or phase imbalance correction, including phasing and prephasing correction.

E50: Determining sequences of the first barcode sequence and the second barcode sequence according to an intensity difference between the sequencing signals.

This step includes: classifying the sequencing signals into sequence results of the first barcode sequence and the second barcode sequence correspondingly, according to an intensity difference between the sequencing signals, which includes:

i. determining, for each cycle of sequencing, intensities of the sequencing signals.

This step includes making a correspondence for the signals generated by sequencing according to the position. For each position, the sequencing signals at this position in each cycle of sequencing will include at least two sequencing signals with different intensities, that is, among channels the sequencing signals obtained in, at least two channels at this position present sequencing signals, or in the case of one channel collects signals for multiple times, there are sequencing signals appeared twice with different intensities. In one embodiment, the sequencing signal is an optical signal, for example, such as a fluorescent signal.

It should be understood that, in this embodiment, it is required to, before dual-indexing sequencing, determine the relative content ranking of the first barcode and the second barcode that could be extendable by the difference in sequencing signals presented in sequencing. Such a step includes determining the signal intensities of the first barcode and the second barcode based on the intensities of sequencing signals obtained from this position in at least one cycle of sequencing. Illustratively, taking a four-channel as an example, if two of sequencing signals obtained by the four-channel in images generated by multiple cycles of sequencing have intensities at a relatively constant ratio, such as 7:3, it is determined that a ratio of the copy number of the two extendable barcodes at this position is approximately 7:3 based on the signal intensities.

It should be understood that the intensity referred to in the step may be an original intensity of the fluorescence image or a corrected signal intensity. As previously described, the correction may be crosstalk correction and/or phase imbalance correction.

ii. Sorting, in ascending or descending order, the sequencing signals in each cycle of sequencing according to the relative signal intensities.

Specifically, the step ii includes: sorting, according to the intensity of the sequencing signals, the sequencing

signals in each cycle of sequencing based on the intensity of each sequencing signal at each position. In one embodiment, the sequencing signals in each cycle of sequencing are sorted in descending of the relative signal intensities. In other embodiment, the sequencing signals in each cycle of sequencing are sorted in ascending of the relative signal intensities.

Taking fluorescence intensity as an example, both the A base channel and T base channel show fluorescence signals in the fluorescence image collected by one cycle of sequencing, and the fluorescence signal intensity of the two channels is of 7:3. At this case, the sequencing signals obtained from the two channels are sorted according to the order of fluorescence signal intensity from high to low or from low to high. For example, according to the order of the fluorescence signal intensity from high to low, the sequencing signals are ranked as a first sequencing signal and a second sequencing signal.

It should be understood that, in this step, it is able to know the base type corresponding to a high and low signal intensity by sorting sequencing signals in each cycle of sequencing. Continuing with the above example, at the first position containing two nucleic acid templates where the copy number of the first nucleic acid template to that of the second nucleic acid template is about 7:3, both the A base channel and T base channel show fluorescence signals in the fluorescence image collected by one cycle of sequencing, and the fluorescence signal intensity of the two channels is of 7:3. At this case, the first sequencing signal corresponds to the base type of A, and, correspondingly, the base introduced onto the first nucleic acid template in this cycle of sequencing is A; while the second sequencing signal corresponds to the base type of T, and, correspondingly, the base introduced onto the second nucleic acid template in this cycle of sequencing is T.

It should be understood that in some embodiments, there may be cases where the two barcode are introduced with the same nucleotide type in one cycle of sequencing, in which case, this position will exhibit a sequencing signal in one channel, or this position will exhibit a sequencing signal in one channel that is much higher than that of other channels, so that with such a signal intensity, it can be determined that this cycle of sequencing introduces only one type of nucleotide at this position.

iii. Determining the sequencing result for the first barcode sequence and the second barcode sequence each by concatenating the sequencing signal from each cycle of sequencing based on a sorting result.

In one embodiment, determining the sequencing result for each nucleic acid template by concatenating the sequencing signal from each cycle of sequencing based on a sorting result of the sequencing signal includes:

determining the sequencing signals with the same sorting rank in each cycle of sequencing based on the sorting result of the sequencing signal, where the sequencing signals are from the channels;
determining the sequencing signals with the same sorting rank as the sequencing signals derived from one barcode, where the sequencing signals are from the channels; and
concatenating the sequencing signal from each cycle of sequencing derived from one barcode, and obtaining the sequencing result of each barcode based on the concatenated sequencing signal.

Therefore, after sequencing signals obtained in each cycle of sequencing are sorted, a sequence of the nucleic acid template with the highest copy number or the lowest copy number in this position can be obtained by sequentially concatenating base types corresponding to the sequencing signals ranked first in each cycle of sequencing. By analogy, sequences of the nucleic acid templates with reduced or increased copy number can be obtained in turn according to the sequence signal from strong to weak or from weak to strong.

Further, the method includes:
determining a sample source of the insert fragment according to the sequences of the first barcode sequence and the second barcode sequence.

The solution of the present disclosure will be explained with reference to the following Examples. Those skilled in the art will appreciate that the following examples are merely illustrative of the disclosure and should not be construed as limiting the scope of the disclosure. If specific techniques or conditions are not indicated in the examples, the techniques or conditions described in the literature in the art or the product specification are followed. Reagents or instruments used without manufacturer indication are commercially available conventional products.

**Example 1: dual-indexing sequencing on one position**

In this Example, two indexes (used interchangeably with barcodes) were simultaneously sequenced in one cycle of sequencing with the sequencing method of the present disclosure. Specifically, in this Example, a standard library was used as a target library to be sequenced, where both ends of a nucleic acid molecule in the library respectively contained two index sequences, index1 (17) and index2 (15). The Indexes sequenced in the Example includes 8 pairs of dual-barcodes as shown in Table 1 below.

Table 1

| ID | index1 (17) sequence | Index2 (15) sequence |
|---|---|---|
| UDI 1 | GGCATT | CTAGCT |
| UDI 2 | AATGCC | TCGATC |
| UDI 3 | AGCCTC | AGTAGA |
| UDI 4 | GATTCT | GACGAG |
| UDI 5 | TCGTAG | AGACTT |
| UDI 6 | CTACGA | GAGTCC |
| UDI 7 | TGCGGC | CCTCGG |
| UDI 8 | CATAAT | TTCTAA |

After constructing the sequencing library and sequencing the standard, sequencing on dual-indexing was performed as follows.

1.1 A primer mix (mix 1) containing index1 sequencing primer (BC1), index2 sequencing primer (BC2), index sequencing blocking molecule 1 (BC1-ddC) which has the same sequence as BC1 but is not extendable at the 3' end) was prepared. Primer sequences of BC1, BC2 and BC1-ddC are shown below, a molar ratio of which is 0.5: 1: 0.5, with the total primer concentration of 1 umol/L.

BC3 sequence: 5'- AATGATACGGCGACCACCGAGATCTACAC (SEQ ID NO: 1)
BC1 sequence: 5'-GATCGGAAGAGCACACGTCTGAACTCCAGTCAC (SEQ ID NO: 2)
BC1-ddC sequence: 5'-GATCGGAAGAGCACACGTCTGAACTCCAGTCA-ddC (SEQ ID NO: 3)

1.2 Hybridization sequencing was performed with mix 1 on a Geno Lab M sequencer, and sequencing was performed for 6 cycles of base extension.

1.3 Sequencing image data was collected and analyzed to draw a crosstalk plot based on signals of four colors and to base call.

**Comparative Example**

The comparative Example differs from Example 1 in step 1.2, where index1 (17) was sequenced by hybridized BC1, and the sequencing involved 12 cycles of base extension.

The method in the comparative Example generated sequencing signals only for index1 (I7), and a crosswalk plot is also drawn for base call.

FIG. 8 is a crosstalk plot obtained with control data from hybridizing BC1 alone in comparative Example. In this plot, each dot represents a signal on the chip surface. Taking the "AT" plot at the top right as an example, a group of data points with green signal (A) significantly higher than orange signal (T) forms an "arm" in which any point could be identified as an "A" base. A group of data points with orange signal (T) significantly higher than green signal (A) forms another "arm" in which any point could be identified as a "T" base. The two arms representing the A and T signals form an angle of about 70°, reflecting a certain crosstalk from the green and orange signals in the detection. Fewer data points between the two arms indicates that most fluorescence data comes from a single sequencing reaction rather than two sequencing reactions performed at one position.

FIG. 9 is a crosstalk plot obtained by sequencing two indexes simultaneously using BC1 and BC2 in Example 1. Similarly, each dot represents a signal on the chip surface. Example 1 used double primers at a specific ratio to sequence the two indexes, there are therefore 4 arms in partial crosstalk plot, a mixed signal from the first base of index1 and index2. Taking the "AT" plot at the top right as an example, the two arms representing the A and T signals still form an angle of about 70°, but there are two new arms between the A and T signals, which respectively represent A+0.5T (i.e. containing A signal and T signal at an intensity ratio of 1:0.5) and T+0.5A (i.e. containing T signal and A signal at an intensity ratio of 1:0.5). The new two arms show that two different bases or nucleotides reacted at one position in a ratio of 1:0.5 (this ratio is determined by the concentration of BC1 and BC2 in mix 1) in a cycle of base extension. Because the concentration of BC1 and BC2 in mix 1 is 0.5:1, the signal intensity of the two indexes involved in each cycle of base extension also presents as strong and weak, and the signal generated by index1 is always 0.5 times that generated by index3. Accordingly, it can be determined for the A+0.5T signal that the A signal is from index3 (i5) and the T signal is from index1 (i7). The other multi-arm cases in the plot may be read in terms of base signal with a similar way.

It is worth noting that the four arms appear only in the "AT","CG", and "CT" plots in FIG. 8, rather than all plots. This is because there are only three different base combinations of A-T, C-G and C-T in eight pairs of UDIs when reading the first base of index1 and index2 simultaneously. Specifically, the three different base combinations are G-C combination of UDI1

and UDI6, A-T combination of UDI2 and UDI5, and C-T combination of UDI7 and UDI8, and the same base is of the same color and does not need to be distinguished, such as A-A in UDI3. From this, it could be seen that crosstalk of each multi-arm corresponds to exactly one base combination; if there are no mixed bases in UDI, multi-arm such as A-G mixed bases will not appear in the crosstalk, which indicates the feasibility of the sequencing method provided the Examples.

It can be seen that with the sequencing method provided in the Examples of the present disclosure, each cycle of base extension can realize simultaneous calling on two bases, thereby doubling the sequencing speed.

**Example 2**

In this Example, two indexes (used interchangeably with barcodes) were simultaneously sequenced in one cycle of sequencing with the sequencing method of the present disclosure. Specifically, in this Example, a standard library was used as a target library to be sequenced, where both ends of a nucleic acid molecule in the library respectively contained two index sequences, index3 and index4, sequences of which are AATGCC and GGCATT, respectively.

After constructing the sequencing library and sequencing the standard, sequencing on dual-indexing was performed as follows. A primer mix (mix 2) containing index3 sequencing primer and index4 sequencing primer at a molar ratio of 3: 1 was prepared, with the total primer concentration of 1 umol/L. Hybridization sequencing was performed with mix 2 on a Geno Lab M sequencer, and sequencing was performed for 6 cycles of base extension. Sequencing image data was collected, and results are shown in FIG. 10, in which the abscissa represents the number of cycles and the ordinate represents brightness. In each cycle, the four channels A, T, G and C are represented from left to right.

In fluorescence images obtained in the first cycle of base extension, the result shows that the intensity ratio of the four bases A, T, G and C is 8.2: 0: 2.6: 0, thus recognizing base A and base G with the sequencing signal intensity ratio of about 3:1.

In fluorescence images obtained in the second cycle of base extension, the result shows that the intensity ratio of the four bases A, T, G and C is 4.5: 1: 1.8 :0, thus recognizing base A and base G with the sequencing signal intensity ratio between 2:1 to 3:1 from two dominant sequencing signals.

In fluorescence images obtained in the third cycle of base extension, the result shows that the intensity ratio of the four bases A, T, G and C is 0: 5.2: 0: 2.3, thus recognizing base T and base C with the sequencing signal intensity ratio between 2:1 to 3:1 from two dominant sequencing signals.

In fluorescence images obtained in the fourth cycle of base extension, the result shows that the intensity ratio of the four bases A, T, G and C is 2: 0: 6: 0, thus recognizing base G and base A with the sequencing signal intensity ratio of about 3:1.

In fluorescence images obtained in the fifth cycle of base extension, the result shows that the intensity ratio of the four bases A, T, G and C is 0: 2: 0: 6, thus recognizing base C and base T with the sequencing signal intensity ratio of about 3:1.

In fluorescent images obtained in the sixth cycle of base extension, the result shows that the intensity ratio of the four bases A, T, G and C is 0: 2: 0: 6, thus recognizing base C and base T with the sequencing signal intensity ratio of about 3:1.

It can be found from the fluorescent images obtained in the base extension that the double signal position contains two nucleic acid templates, and in each cycle of base extension, the sequencing signal of the two nucleic acid templates is about 3:1, which indicates that the number of molecules of the two nucleic acid templates is about 3:1. A nucleic acid template with more molecules generates sequencing signals with higher intensity in the image of base extension, and by concatenating bases identified by the sequencing signals with higher intensity in the image obtained in each cycle of base extension and similarly, by a nucleic acid template with more molecules generates sequencing signals with higher intensity in the image of base extension, sequences of two nucleic acid templates are obtained as AATGCC and GGCATT, which are consistent with actuality.

**Example 3**

In this Example, two indexes (used interchangeably with barcodes) were simultaneously sequenced in one cycle of sequencing with the sequencing method of the present disclosure. Specifically, in this Example, a standard library was used as a target library to be sequenced, where two ends of a nucleic acid molecule in the library separately contained three index sequences, index5, index6 and index7, sequences of which are TCGTAG, CATAAT and GATTCT, respectively.

After constructing the sequencing library and sequencing the standard, sequencing on dual-indexing was performed as follows. A primer mix (mix 3) containing index5 sequencing primer, index6 sequencing primer and index7 sequencing primer at a molar ratio of 4: 2: 1 calculated with the total ratio of the four channel signals being 7 was prepared, with the total primer concentration of 1 umol/L. Hybridization sequencing was performed with mix 3 on a Geno Lab M sequencer, and sequencing was performed for 6 cycles of base extension. Sequencing image data was collected, and results are shown in FIG. 11, in which the abscissa represents the number of cycles and the ordinate represents

brightness. In each cycle, the four channels A, T, G and C are represented from left to right.

In fluorescence images obtained in the first cycle of base extension, the result shows that the intensity ratio of the four bases A, T, G and C is 0: 3.0: 6.3: 1.6, thus recognizing base T, base C and base G with the sequencing signal intensity ratio of about 4: 2: 1. From the first cycle of base extension it could be inferred that the triple signal position contains three nucleic acid templates, and in at least one cycle of base extension, the sequencing signal of the three nucleic acid templates is about 4: 2: 1, which indicates that the number of molecules of the three nucleic acid templates is about 4: 2: 1.

In fluorescence images obtained in the second cycle of base extension, the result shows that the intensity ratio of the four bases A, T, G and C is 4.6: 0: 0: 5.9, thus recognizing base C and base A with the sequencing signal intensity ratio of about 4: 3. Combined with the base intensity ratio obtained in the first cycle of base extension, it is inferred that both two templates with lower molecular number are identified with base A in this cycle of base extension.

In fluorescence images obtained in the third cycle of base extension, the result shows that the intensity ratio of the four bases A, T, G and C is 0: 4.3: 6.1: 0, thus recognizing base G and base T with the sequencing signal intensity ratio of about 4: 3. Combined with the base intensity ratio obtained in the first cycle of base extension, it is inferred that both two templates with lower molecular number are identified with base T in this cycle of base extension.

In fluorescence images obtained in the fourth cycle of base extension, the result shows that the intensity ratio of the four bases A, T, G and C is 2.8: 7.1: 0: 0, thus recognizing base Tand base A with the sequencing signal intensity ratio of about 5: 2. Combined with the base intensity ratio obtained in the first cycle of base extension, it is inferred that the templates with the highest number of molecules and the lower number of molecules are both identified with base T in this cycle of base extension.

In fluorescence images obtained in the fifth cycle of base extension, the result shows that the intensity ratio of the four bases A, T, G and C is 8.3: 0: 0: 1.3, thus recognizing base A and base C with the sequencing signal intensity ratio of about 6: 1. Combined with the base intensity ratio obtained in the first cycle of base extension, it is inferred that the templates with the highest number of molecules and the second-highest number of molecules are both identified with base A in this cycle of base extension.

In fluorescence images obtained in the sixth cycle of base extension, the result shows that the intensity ratio of the four bases A, T, G and C is 0: 4.5: 6.2: 0, thus recognizing base G and base T with the sequencing signal intensity ratio of about 4: 3. Combined with the base intensity ratio obtained in the first cycle of base extension, it is inferred that both two templates with lower molecular number are identified with base T in this cycle of base extension.

In this Example, the triple signal position contains three nucleic acid templates, and in at least one cycle of base extension, the sequencing signal of the three nucleic acid templates is about 4: 2: 1, indicating that the number of molecules of the three nucleic acid templates is about 4: 2: 1. A nucleic acid template with more molecules generates sequencing signals with higher intensity in the image of base extension, and by concatenating bases identified by the sequencing signals with higher intensity in the image obtained in each cycle of base extension and similarly, by a nucleic acid template with more molecules generates sequencing signals with higher intensity in the image of base extension, sequences of two nucleic acid templates are obtained as AATGCC and GGCATT, which are consistent with actuality.

Reference throughout this specification to "an embodiment", "some embodiments", "one embodiment", "another example", "an example", "a specific example" or "some examples" means that a particular feature, structure, material, or characteristic described in connection with the embodiment or example is included in at least one embodiment or example of the present disclosure. Thus, the appearances of the phrases such as "in some embodiments", "in one embodiment", "in an embodiment", "in another example", "in an example", "in a specific example" or "in some examples", in various places throughout this specification are not necessarily referring to the same embodiment or example of the present disclosure. Furthermore, the particular features, structures, materials, or characteristics may be combined in any suitable manner in one or more embodiments or examples.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

**1.** A sequencing method, comprising:

detecting sequencing signals at at least one assigned position on a solid support during sequencing, signals of the assigned position at least comprising two sequencing signals; and
acquiring base types at the assigned position based on a difference between the sequencing signals, thereby achieving sequencing at the assigned position.

2. The sequencing method according to claim 1, wherein the solid support comprises a plurality of positions, each position is immobilized with a plurality of nucleic acid templates,

the plurality of nucleic acid templates are non-identical; or
the plurality of nucleic acid templates are identical, and the plurality of nucleic acid template as identical have multiple target nucleic acid sequences.

3. The sequencing method according to claim 2, wherein the positions comprise a first position, the first position comprises n different nucleic acid templates, wherein n is an integer greater than or equal to 2.

4. The sequencing method according to claim 3, wherein the n nucleic acid templates at the first position are of different copy numbers.

5. The sequencing method according to claim 4, wherein the n nucleic acid templates at the first position are obtained by:

providing the solid support, bound an surface thereon n probes having different sequences, wherein the n probes have different numbers;
amplifying hybrids obtained by hybridizing the n different nucleic acid templates onto the surface of the solid support, wherein the n different nucleic acid templates hybridize with the n different probes respectively, such that 2 to n different nucleic acid templates bind to one position with different numbers, and designating this one position as the first position.

6. The sequencing method according to claim 5, wherein for the n different probes at the first position,

a difference in a number between different probes is at least greater than or equal to 10% of a total number of the n probes, or
a difference in a molar concentration between different probes is at least greater than or equal to 10% of a total molar concentration of the n probes.

7. The sequencing method according to claim 6, wherein the difference in the number or the molar concentration between different probes is, respectively, at least greater than or equal to 20% of the total number or the total molar concentration of the n probes.

8. The sequencing method according to any one of claims 4 to 7, wherein the n different nucleic acid templates comprise multiple sequence units to be sequenced with different numbers, identical nucleic acid templates have the same sequences of the sequence units to be sequenced, and different nucleic acid templates have different sequences of the sequence units to be sequenced.

9. The sequencing method according to claim 8, wherein the n nucleic acid templates at the first position are of the same or different copy numbers.

10. The sequencing method according to claim 9, wherein for the n different nucleic acid templates, each of the sequence units to be sequenced is provided with a primer binding site.

11. The sequencing method according to claim 10, wherein a difference in a number of the sequence units to be sequenced between the n different nucleic acid templates is at least greater than or equal to 10% of a total number of the sequence units to be sequenced.

12. The sequencing method according to claim 11, wherein the difference in the number of the sequence units to be sequenced between the n different nucleic acid templates is at least greater than or equal to 20% of a total number of the sequence units to be sequenced.

13. The sequencing method according to any one of claims 4 to 12, wherein the n is 2 to 4.

14. The sequencing method according to any one of claims 3 to 13, wherein detecting sequencing signals at an assigned position on a solid support during sequencing, the assigned position at least comprising two sequencing signals comprises:
performing multiple sequencing cycles on the nucleic acid templates at the first position, to acquire the sequencing

signals in each channel for the first position.

**15.** The sequencing method according to claim 14, wherein acquiring base types at the assigned position based on a difference between the sequencing signals, thereby achieving sequencing at the assigned position comprises: classifying the sequencing signal at the first position into a sequence result of corresponding nucleic acid template, according to an intensity difference between the sequencing signals in each channel.

**16.** The sequencing method according to claim 15, wherein classifying the sequencing signal at the first position into a sequence result of corresponding nucleic acid template, according to an intensity difference between the sequencing signals in each channel comprises:

determining, for each cycle of sequencing, the number and intensity of the sequencing signals in respective channels for the first position;
sorting, in ascending or descending order, the sequencing signals in each cycle of sequencing according to the intensity of the sequencing signals; and
determining the sequencing result for each nucleic acid template by concatenating the sequencing signal from each cycle of sequencing based on a sorting result.

**17.** The sequencing method according to claim 16, wherein determining the sequencing result for each nucleic acid template by concatenating the sequencing signal from each cycle of sequencing based on a sorting result comprises:

determining the sequencing signals with the same sorting rank in each cycle of sequencing based on the sorting result;
determining the sequencing signals with the same sorting rank as the sequencing signals from one nucleic acid template; and
concatenating the sequencing signals from one nucleic acid template in each cycle of sequencing, and obtaining the sequencing result of the nucleic acid template based on the concatenated sequencing signal.

**18.** The sequencing method according to any one of claims 2 to 17, wherein the sequencing signal is an optical signal.

**19.** The sequencing method according to any one of claims 2 to 18, wherein the positions comprise a second position in which the nucleic acid templates are copies of an identical nucleic acid molecule.

**20.** The sequencing method according to claim 19, wherein the second position comprises m different target nucleic acid sequences, wherein m is an integer greater than or equal to 2.

**21.** The sequencing method according to claim 20, wherein each nucleic acid template comprises m distinct primer hybridization sites, the m different target nucleic acid sequences are ligated to 3' ends of the m distinct primer hybridization sites, respectively.

**22.** The sequencing method according to claim 21, wherein the m is 2 to 4.

**23.** The sequencing method according to claim 21 or 22, wherein the nucleic acid template at the second position is hybridized to m primers at m distinct primer hybridization sites, and at the same second position, the amounts of the m hybridized primers differ.

**24.** The sequencing method according to claim 23, wherein at least m-1 distinct primer hybridization sites of the nucleic acid template at the second position bind a non-extendable blocking molecule.

**25.** The sequencing method according to claim 24, wherein the blocking molecule is a nucleic acid primer whose 3' end is non-extendable.

**26.** The sequencing method according to claim 25, wherein the second position is obtainable by:

amplifying hybrids obtained by hybridizing the nucleic acid templates onto the surface of the solid support; and
introducing onto the solid support the m primers respectively hybridized to the m primer hybridization sites and m-1 blocking molecules respectively bound to the m-1 primer hybridization sites, such that the nucleic acid templates at one or more positions are hybridized to the primers at distinct primer hybridization sites with different

amounts, and designating each of the one or more positions as a second position.

**27.** The sequencing method according to claim 25, wherein the second position is obtained by:

amplifying hybrids obtained by hybridizing the nucleic acid templates onto the surface of the solid support; and introducing onto the solid support the m primers with different concentrations respectively bound to the m primer hybridization sites, such that the nucleic acid templates at one or more positions are hybridized to the primers at distinct primer hybridization sites with different amounts, and designating each of the one or more positions as a second position.

**28.** The sequencing method according to claim 25, wherein the second position is obtained by:

amplifying hybrids obtained by hybridizing the nucleic acid templates onto the surface of the solid support; and introducing onto the solid support the m primers with different lengths respectively bound to the m primer hybridization sites, such that the nucleic acid template at one or more positions are hybridized to the primers at distinct primer hybridization sites with different amounts, and designating each of the one or more positions as a second position.

**29.** The sequencing method according to any one of claims 19 to 28, wherein each nucleic acid template at the second position comprises:

a first primer hybridization site;
a first target molecule, ligated with a 3' end of the first primer hybridization site;
a second primer hybridization site, located on the side distal to the first target molecule; and
a second target molecule, ligated with a 3' end of the second primer hybridization site.

**30.** The sequencing method according to claim 29, wherein the first target molecule comprises a first barcode sequence and a first insert fragment, the second target molecule comprises a second barcode sequence and a second insert fragment, the first barcode sequence and the second target molecule are of different nucleic acid sequences.

**31.** The sequencing method according to claim 30, wherein the first insert fragment is complementary to the second insert fragment to form a complementary sequence.

**32.** The sequencing method according to any one of claims 29 to 31, wherein the nucleic acid templates at the second position are obtained by:

a. ligating a first adapter comprising the first primer hybridization site to an end of the first target molecule;
b. ligating, via a second adaptor comprising the second primer hybridization site, the second target molecule to a product of step a; and
c. amplifying a product of step b.

**33.** The sequencing method according to any one of claims 29 to 31, wherein the nucleic acid templates at the second position are obtained by:

i. ligating a first adapter and a second adapter respectively to two 5' terminals of a double-strand target molecule, the first adapter comprising the first primer hybridization site;
ii. ligating, via a cyclizing adapter comprising the second primer hybridization site, the first adapter to a 3' terminal of another target molecule;
iii. subjecting a product of step ii to single-strand transformation, to obtain a single-strand nucleic acid template; and
iv. amplifying the nucleic acid template.

**34.** The sequencing method according to any one of claims 19 to 33, wherein detecting sequencing signals at an assigned position on a solid support during sequencing comprises:
performing multiple sequencing cycles on the nucleic acid templates at the second position, to acquire the sequencing signals in each channel for the second position.

**35.** The sequencing method according to claim 34, wherein acquiring base types at the assigned position based on a

difference between the sequencing signals, thereby achieving sequencing at the assigned position comprises: classifying the sequencing signal at the second position into a sequence result of corresponding target nucleic acid sequence, according to an intensity difference between the sequencing signals in each channel.

**36.** The sequencing method according to claim 35, wherein classifying the sequencing signal at the second position into a sequence result of corresponding target nucleic acid sequence, according to an intensity difference between the sequencing signals in each channel comprises:

determining a value of the m for the second position and relative signal intensities attributable to each of the m target nucleic acid sequences, based on a number of the channels having the sequencing signals and an signal intensity in each channel acquired from the second position in at least one cycle of sequencing;
sorting the sequencing signals in each cycle of sequencing according to the relative signal intensities in channels for the second position in each cycle of sequencing; and
determining the sequencing result for each target nucleic acid sequence by concatenating the sequencing signal from each cycle of sequencing based on a sorting result.

**37.** The sequencing method according to claim 36, wherein determining the sequencing result for each nucleic acid template by concatenating the sequencing signal from each cycle of base extension based on a sorting result comprises:

determining the sequencing signals with the same sorting rank in each cycle of sequencing based on the sorting result;
determining the sequencing signals with the same sorting rank as the sequencing signals from one target nucleic acid sequence; and
concatenating the sequencing signal from one target nucleic acid sequence in each cycle of sequencing, and obtaining the sequencing result of the target nucleic acid sequence based on the concatenated sequencing signal.

**38.** The sequencing method according to any one of claims 19 to 37, wherein the sequencing signal is an optical signal.

**39.** The sequencing method according to any one of claims 1 to 38, wherein the positions comprise a third position in which the nucleic acid templates are identical and each nucleic acid template has one identical primer hybridization site.

**40.** The sequencing method according to any one of claims 2 to 39, wherein at the same one position, at least two of different target nucleic acid sequences generate different sequencing signals in at least one cycle of sequencing.

**41.** The sequencing method according to any one of claims 1 to 40, wherein the solid support is a substrate with a micropore array, and each position corresponds to a location a micropore at.

**42.** A sequencing method, comprising:

constructing a sequencing library, wherein the sequencing library comprises a plurality of nucleic acid molecules, each nucleic acid molecule comprises:

a first barcode, comprising a first primer hybridization site and a first barcode sequence ligated with a 3' end of the first primer hybridization site;
a second barcode, comprising a second primer hybridization site and a second barcode sequence ligated with a 3' end of the second primer hybridization site;
a target molecule, ligated between the first barcode and the second barcode, comprising: a third primer hybridization site and an insert fragment ligated with a 3' end of the third primer hybridization site, the target molecule is ligated with a 3' end of the first barcode via the third primer hybridization site, and the target molecule is ligated with a 5' end of the second barcode via the insert fragment;

amplifying the nucleic acid molecules of the sequencing library after immobilized onto a surface of the solid support, to form a position with a nucleic acid molecule cluster;
introducing a first primer, a blocking molecule and a second primer to hybridize with the nucleic acid molecule of the nucleic acid molecule cluster, wherein the first primer and the blocking molecule are hybridized to the first primer hybridization site, the second primer is hybridized to the second primer hybridization site, and the blocking

molecule is of a non-extendable 3' end;
performing multiple sequencing cycles on the first barcode and the second barcode simultaneously, to acquire sequencing signals in each channel in each cycle of sequencing; and
determining sequences of the first barcode sequence and the second barcode sequence according to an intensity difference between the sequencing signals.

**43.** The method according to claim 42, wherein before introducing the first primer, the blocking molecule and the second primer to hybridize with the nucleic acid molecule, the method further comprises:
introducing a third primer to hybridize with the nucleic acid molecule to sequence the insert fragment, wherein the third primer is hybridized to the third primer hybridization site.

**44.** The method according to claim 42 or 43, wherein the method further comprises:
determining a sample source of the insert fragment according to the sequences of the first barcode sequence and the second barcode sequence.

**45.** The method according to claim 42 or 43, wherein a usage ratio of the blocking molecule to the first primer is 2:8-8:2.

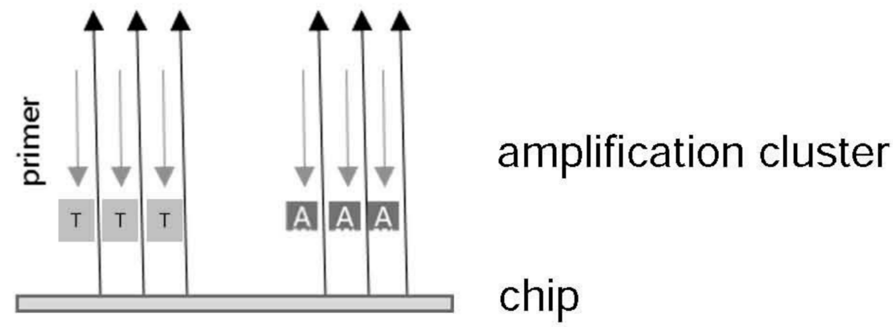


FIG.1

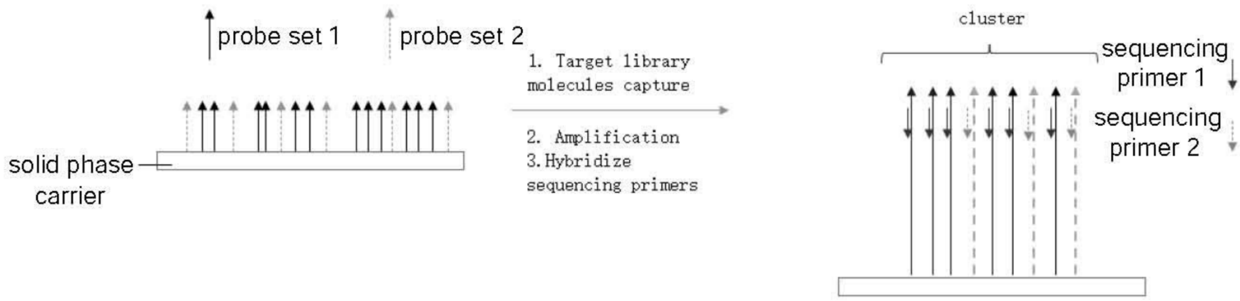


FIG.2

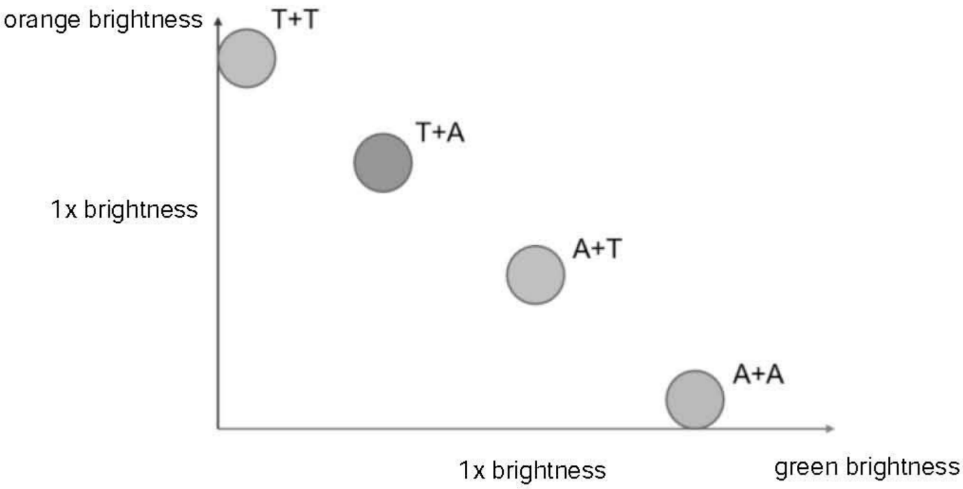


FIG.3

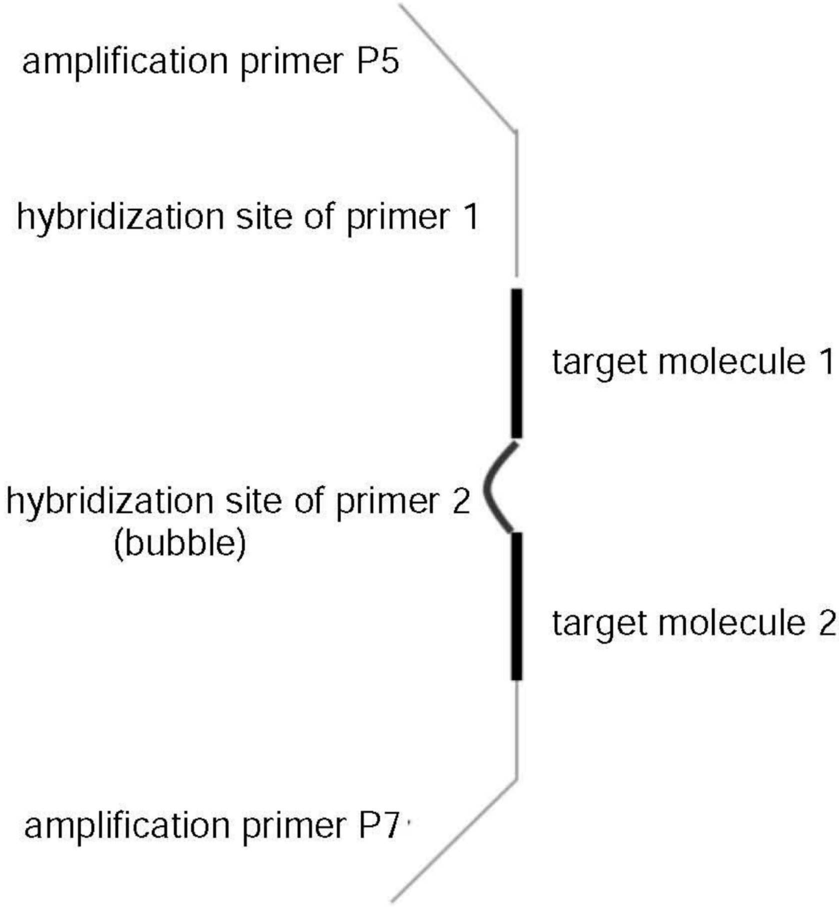
amplification primer P5
hybridization site of primer 1
target molecule 1
hybridization site of primer 2
(bubble)
target molecule 2
amplification primer P7

FIG.4

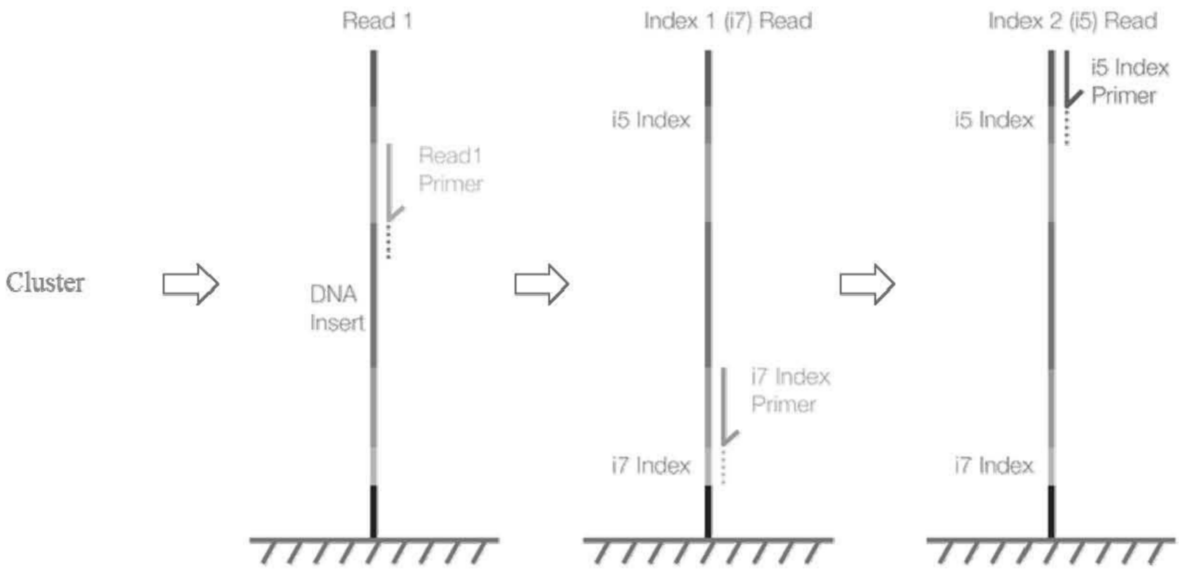
Read 1
Index 1 (i7) Read
Index 2 (i5) Read
Read1
Primer
Cluster
DNA
Insert
i5 Index
i7 Index
Primer
i7 Index
i5 Index
Primer
i5 Index
i7 Index

FIG.5

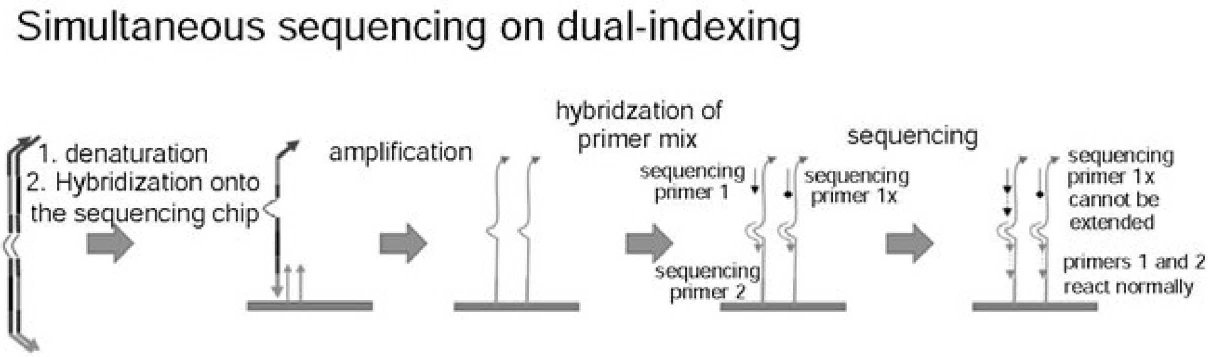


FIG.6

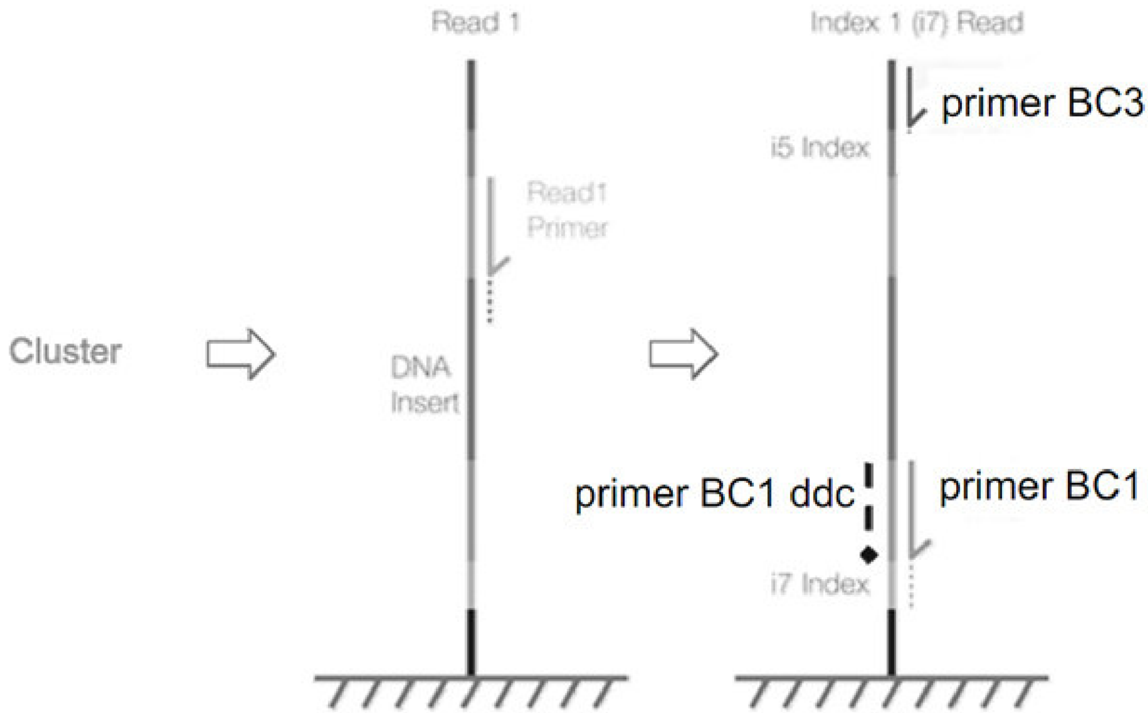


FIG.7

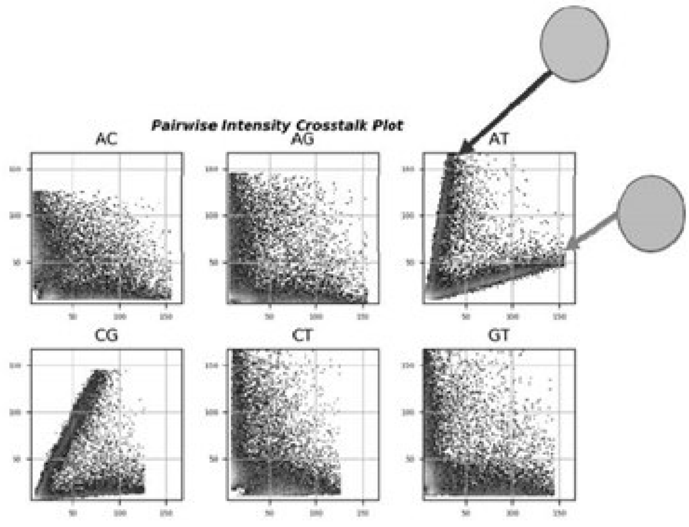


FIG.8

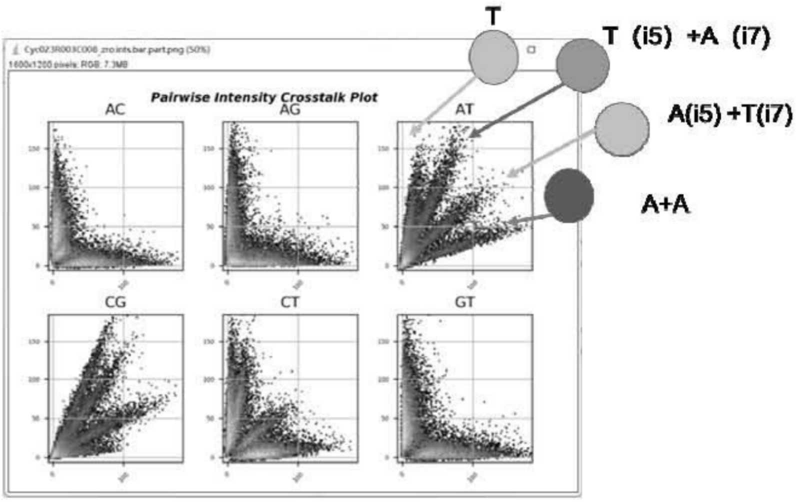


FIG.9

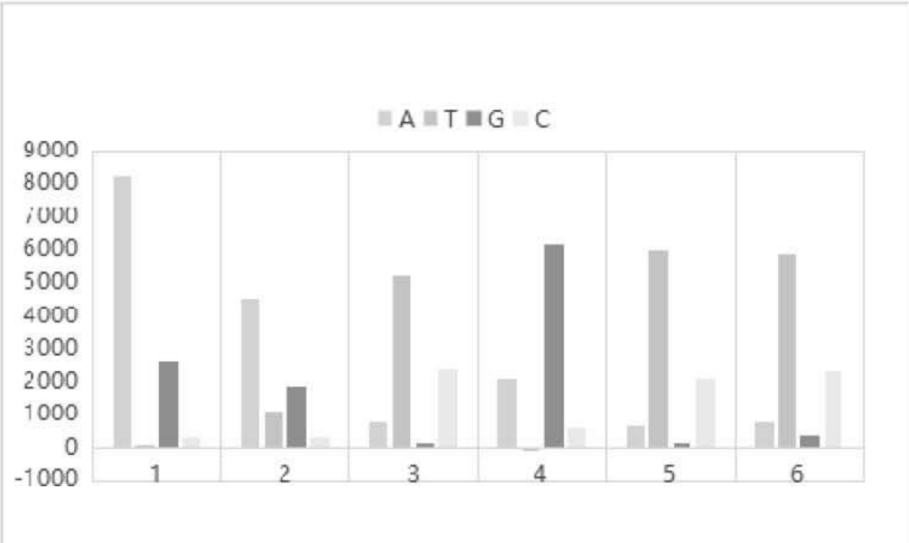


FIG.10

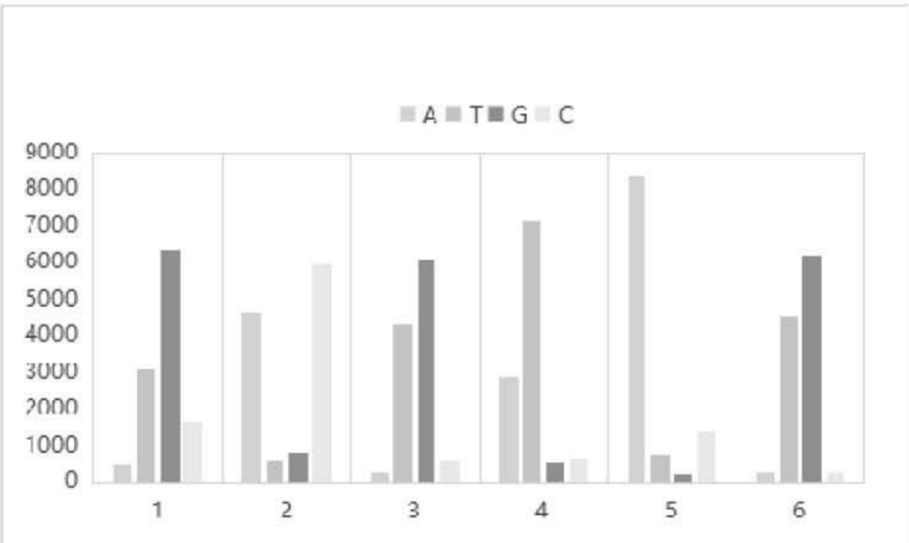


FIG.11

## INTERNATIONAL SEARCH REPORT

International application No.
**PCT/CN2024/075588**

**A. CLASSIFICATION OF SUBJECT MATTER**

C12Q 1/6874(2018.01)i; C12Q 1/6806(2018.01)i; C40B 50/06(2006.01)i

According to International Patent Classification (IPC) or to both national classification and IPC

**B. FIELDS SEARCHED**

Minimum documentation searched (classification system followed by classification symbols)

IPC: C12Q, C40B

Documentation searched other than minimum documentation to the extent that such documents are included in the fields searched

Electronic data base consulted during the international search (name of data base and, where practicable, search terms used)

CNABS, VEN, CNKI, CNTXT, USTXT, EPTXT, PUBMED, ISI WEB OF SCIENCE: 深圳市真迈生物科技有限公司, 李林森, 孙雷, 测序, 通量, 核酸, 固相载体, 通道, 信号, 差异, 标签, sequencing, throughput, nucleic acids, solid support, channel, signal, difference, gap, compare, tag

**C. DOCUMENTS CONSIDERED TO BE RELEVANT**

| Category* | Citation of document, with indication, where appropriate, of the relevant passages | Relevant to claim No. |
|---|---|---|
| X | US 2019352707 A1 (ULTIMA GENOMICS, INC.) 21 November 2019 (2019-11-21) claims 1-53 | 1-13 |
| Y | US 2019352707 A1 (ULTIMA GENOMICS, INC.) 21 November 2019 (2019-11-21) claims 1-53 | 14-45 |
| Y | US 2018282800 A1 (BGI SHENZHEN) 04 October 2018 (2018-10-04) claims 1-29, and description, paragraphs 68 and 92 | 14-45 |
| Y | WO 2022247555 A1 (GENEMIND BIOSCIENCES CO., LTD.) 01 December 2022 (2022-12-01) claims 1-29 | 20-45 |
| A | CN 114550818 A (CYGNUS BIOSCIENCES (BEIJING) CO., LTD.) 27 May 2022 (2022-05-27) claims 1-11 | 1-45 |
| A | US 2022002798 A1 (BGI SHENZHEN) 06 January 2022 (2022-01-06) claims 1-22 | 1-45 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 May 2024** | **23 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/075588**

**C. DOCUMENTS CONSIDERED TO BE RELEVANT**

| Category* | Citation of document, with indication, where appropriate, of the relevant passages | Relevant to claim No. |
|---|---|---|
| A | CN 114196744 A (CYGNUS BIOSCIENCES (BEIJING) CO., LTD.) 18 March 2022 (2022-03-18)<br>claims 1-8 | 1-45 |
| A | CN 102831330 A (BEIJING NOVOGENE BIOINFORMATICS TECHNOLOGY CO., LTD.) 19 December 2012 (2012-12-19)<br>claims 1-15 | 1-45 |

Form PCT/ISA/210 (second sheet) (July 2022)

**INTERNATIONAL SEARCH REPORT**
**Information on patent family members**

International application No.
**PCT/CN2024/075588**

| Patent document cited in search report | | | Publication date (day/month/year) | Patent family member(s) | | | Publication date (day/month/year) |
|---|---|---|---|---|---|---|---|
| US | 2019352707 | A1 | 21 November 2019 | US | 2023313287 | A1 | 05 October 2023 |
| | | | | WO | 2018064297 | A1 | 05 April 2018 |
| US | 2018282800 | A1 | 04 October 2018 | WO | 2016077313 | A1 | 19 May 2016 |
| | | | | US | 10626455 | B2 | 21 April 2020 |
| | | | | DK | 3218519 | T3 | 21 December 2020 |
| | | | | EP | 3218519 | A1 | 20 September 2017 |
| | | | | EP | 3218519 | A4 | 25 July 2018 |
| | | | | EP | 3218519 | B1 | 02 December 2020 |
| WO | 2022247555 | A1 | 01 December 2022 | EP | 4350007 | A1 | 10 April 2024 |
| CN | 114550818 | A | 27 May 2022 | | None | | |
| US | 2022002798 | A1 | 06 January 2022 | WO | 2020113581 | A1 | 11 June 2020 |
| CN | 114196744 | A | 18 March 2022 | | None | | |
| CN | 102831330 | A | 19 December 2012 | | None | | |

Form PCT/ISA/210 (patent family annex) (July 2022)